(19) Europäisches Patentamt
European Patent Office
Office européen des brevets

(11) EP 1 661 993 A1

# (12) EUROPEAN PATENT APPLICATION

(43) Date of publication:
**31.05.2006 Bulletin 2006/22**

(21) Application number: **04028251.9**

(22) Date of filing: **29.11.2004**

(51) Int Cl.:
*C12N 15/12* (2006.01) *C07K 14/47* (2006.01)
*C12N 15/11* (2006.01) *A61K 31/7088* (2006.01)
*C07K 16/18* (2006.01) *A61K 38/00* (2006.01)
*G01N 33/50* (2006.01) *G01N 33/68* (2006.01)
*C12Q 1/68* (2006.01) *A61P 37/06* (2006.01)

(84) Designated Contracting States:
**AT BE BG CH CY CZ DE DK EE ES FI FR GB GR HU IE IS IT LI LU MC NL PL PT RO SE SI SK TR**
Designated Extension States:
**AL HR LT LV MK YU**

(71) Applicant: **Technische Universität München**
**80333 München (DE)**

(72) Inventors:
- **Holzmann, Bernhard**
  **81547 München (DE)**
- **Beer, Sandra**
  **40221 Düsseldorf (DE)**
- **Pfeffer, Klaus**
  **40221 Düsseldorf (DE)**

(74) Representative: **Vossius & Partner**
**Siebertstrasse 4**
**81675 München (DE)**

## (54) SLY1 inhibitors in organ, tissue and cell transplantation

(57) The present invention relates to a pharmaceutical composition comprising an inhibitor/negative regulator/antagonist of SLY-1 (SH3 domain protein expressed in lymphocytes). The use of an inhibitor/negative regulator/antagonist of SLY-1 for the preparation of a pharmaceutical composition for the treatment or prevention of undesired side effects in transplantation of cells, tissues or organs and/or for the treatment of graft-versus-host disease. Furthermore, a method is also described for treating or preventing undesired side effects in transplantation of cells, tissues or organs in a subject comprising administering an inhibitor/negative regulator/antagonist of SLY-1 to mammal in need of such a therapy is provided. The present invention also relates to a method for screening inhibitors or negative regulators of SLY-1 activity or expression comprising the steps of (a) contacting a cell or a non-human organism expressing SLY-1 or capable of expressing SLY-1 with a compound to be tested; (b) determining antigen-receptor signal transductions in the presence of said compound to be tested when compared to a cell not contacted with said compound; and (c) identifying the compound which inhibits SLY-1 activity.

EP 1 661 993 A1

## Description

The present invention relates to a pharmaceutical composition comprising an inhibitor/negative regulator/antagonist of SLY-1 (SH3 domain protein expressed in lymphocytes). The use of an inhibitor/negative regulator/antagonist of SLY-1 for the preparation of a pharmaceutical composition for the treatment or prevention of undesired side effects in transplantation of cells, tissues or organs and/or for the treatment of graft-versus-host disease. Furthermore, a method is also described for treating or preventing undesired side effects in transplantation of cells, tissues or organs in a subject comprising administering an inhibitor/negative regulator/antagonist of SLY-1 to mammal in need of such a therapy is provided. The present invention also relates to a method for screening inhibitors or negative regulators of SLY-1 activity or expression comprising the steps of (a) contacting a cell or a non-human organism expressing SLY-1 or capable of expressing SLY-1 with a compound to be tested; (b) determining antigen-receptor signal transductions in the presence of said compound to be tested when compared to a cell not contacted with said compound; and (c) identifying the compound which inhibits SLY-1 activity.

Immune reactions are triggered by various stimuli including conserved microbial components that activate the innate immune system and cognate antigens that activate T and B lymphocytes in adaptive immunity. CD4 T lymphocytes can differentiate in distinct functional classes that differ in the secretion of effector cytokines and play central regulatory roles in adaptive immune responses. The lineage decisions for differentiation of helper T cells may be influenced by cytokine receptor signaling as well as signals derived from costimulatory receptors (Murphy and Reiner, 2002). $T_H1$ cells produce IFN-$\gamma$ and are responsible for host defense through cell-mediated immunity. Under pathological conditions, $T_H1$ cells may promote autoimmune reactions as well as rejection of allografts (Freedman et al., 2003; Walsh et al., 2004). In contrast, $T_H2$ cells secrete IL-4 as their major effector cytokine and support humoral immune responses. Recently primed CD4 T helper cells migrate to the T cell zone of B cell follicles where they encounter antigen-specific B cells that have also migrate to this area (Garside et al., 1998). CD4 T cells deliver, primarily through CD40, a signal for B cell expansion and antibody isotype switching (Coffey et al., 1998; Renshaw et al., 1994; Xu et al., 1998).

Both B and T lymphocytes use antigen receptor engagement to initiate distinct signal transduction pathways that direct ultimate cellular responses. In recent years, it has become apparent that adaptor molecules regulate the coupling of receptor-proximal events, such as protein tyrosine kinase activation, with end results such as inducible gene expression and cytoskeletal rearrangements. While adaptor molecules possess no intrinsic enzymatic activity, their ability to mediate protein-protein interactions is vital for the integration and propagation of signal transduction cascades in lymphocytes (Kurosaki, 2002; Jordan et al., 2003; Samelson, 2002). Adaptor molecules may contain multiple phosphorylatable residues or proline-rich regions that allow them to be recognized by interaction domains such as SH2 and SH3 on other signaling proteins. Therefore, adaptor molecules are able to coordinate interactions among the signal transduction effector proteins. This characteristic suggests that adaptor molecules act as intracellular scaffolds around which signaling complexes are organized. The use of genetically manipulated mice has shown that adaptor proteins play essential roles in T and B lymphocyte development and in activation of adaptive immune responses (Simeoni et al., 2004).

Recently, the novel adaptor protein SLY1 (SH3 domain protein expressed in lymphocytes) was identified, which contains both SH3 and SAM domains and is specifically expressed in T and B lymphocytes (Beer et al., 2001). Using a phosphopeptide-specific antiserum it was demonstrated that SLY1 is phosphorylated on Ser27 following T or B cell receptor ligation (Astoul et al., 2003). Moreover, inhibitor studies implied that SLY1 represents a target for phosphorylation by PKC or a PKC-dependent protein kinase in antigen receptor-stimulated lymphocytes. These studies therefore defined SLY1 as a target for antigen receptor signal transduction.

The technical problem of the present invention is the provision of means and methods for the alleviation of immunological disorders relating to transplantation of biological material(s) in patients, preferably human patients. The solution to this technical problem is provided in the present invention as characterized in the claims and as illustrated and exemplified herein.

The invention relates to a pharmaceutical composition comprising an inhibitor/negative regulator/antagonist of SLY-1 (SH3 domain protein expressed in lymphocytes).

As documented in the appended examples, *Sly1*-deficient mice were generated to investigate the physiological functions of this novel SH3 and SAM domain containing -protein in T and B cell responses. Most surprisingly, *Sly1*-deficiency impairs lymphoid organ development and antigen receptor-mediated lymphocyte activation. As shown, *Sly1-deficient* mice exhibit severe defects in humoral immune responses. *Sly1*-deficient mice show extended allograft survival a finding which is of high relevance for medical intervention in transplantations and in the amelioration of graft-versus-hosts disease. Thus, SLY1 plays a critical role in adaptive immunity and genetic inactivation of the *Slyl* gene may represent a novel type of X-linked immunodeficiency.

In accordance with this invention, the term "SLY-1" relates to "SH3 domain protein expressed in lymphocyte" as known to the person skilled in the art and also characterized in Beer (2001); Astoul (2003); Beer (2003), Immunobiology 208, 115; or Beer (2004), Clinical and Investigative Medicine, 27(4), No. 4, p2B. Accordingly, SLY is a novel adaptor protein with a SH3 and SAM domain which is exclusively expressed in lymphoid organs. SLY gets phosphorylated upon antigen receptor triggering through a still unknown serine/threonine kinase. As known in the art, SLY-deficient mice show significantly reduced cell numbers in most of the lymphoid organs although the relative percentage of lymphocyte subpopulations remains largely unaltered. Additionally, antigen receptor signalling in B and T cells measured as cytokine production and proliferation is strongly impaired. Basal immunoglobulin levels were reduced an immune responses in vivo were attenuated in SLY-deficient mice. However, as known in the art, impaired cytokine production and proliferation are unspecific reactions to antigen-receptor-stimulation. Accordingly, it is most surprising that a down-regulation of SLY-1 or even a complete inhibition (or knock-out) of SLY-1 expression and/or function leads to significantly prolonged survival of allografts/transplanted tissue. A lower cytokine production- and proliferation is not indicative for the development of alloreactive, cytotoxic T-effector cells and/or the migration of these cells to transplanted tissue in vivo; see also Koretzky (2001), Nat. Rev. Immunol. 1, 95-107.

Therefore, the invention provides for the first medical use of SLY-1 inhibitors/antagonists in the treatment and/or prevention of side effects, like transplant/graft rejection, in transplantation therapy or in the treatment, alleviation and/or prevention of graft-versus-host disease.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease, namely transplant rejection and/or graft-versus-host disease, from occurring in a subject which may be predisposed to the disease; (b) inhibiting the disease, i.e. arresting its development; or (c) relieving the disease, i.e. causing regression of the disease.

The term "inhibitor" comprises competitive, non-competitive, functional and chemical antagonists as described, inter alia, in Mutschler, "Arzneimittelwirkungen" (1986), Wissenschaftliche Verlagsgesellschaft mbH, Stuttgart, Germany. The term "partial inhibitor" in accordance with the present invention means a molecule or substance or compound or composition or agent or any combination thereof that is capable of incompletely blocking the action of agonists through, inter alia, a non-competitive mechanism. It is preferred that said inhibitor alters, modulates and/or prevents activation or function of SLY-1 adaptor protein or the interaction of SLY-1 adaptor proteins with its in vivo binding partner, like protein kinase (CPKc) or PKC-dependent protein kinases and illustrated herein. Preferably, said inhibition leads to partial, preferably complete, standstill. Said standstill may either be reversible or irreversible.

Preferably, the inhibitor/antagonist of a SLY-1 adaptor protein alters, modulates and/or prevents antigen-receptor signal transduction. Further functions which may be altered, modulated or prevented comprise the phosphorylation status of SLY-1 (as discussed below), the proliferation of lymphocytes (an inhibitor/antagonist of SLY-1 leads to a down regulation of proliferation), the production of cytokines (an inhibitor/antagonist of SLY-1 leads to a down regulation of cytokine production, e.g., the production of interleukine-2 and/or interferone-$\gamma$).

In a preferred embodiment of the invention an inhibitor/negative regulator/antagonist to be used in the pharmaceutical composition in the preparation of a medicament or the methods of the invention is selected from the group consisting of small-binding molecules, intracellular-binding receptors, aptamers, intramers, RNAi (double-stranded RNA), siRNA and anti-SLY-1-antisense molecules. Furthermore inhibitors/antagonists are mentioned herein below in context of the inventive screening methods provided herein.

The intracellular binding receptor to be used may be an intracellular antibody, antibody derivatives or an antibody fragment directed against SLY-1. Genetical antibodies or fragments or derivatives thereof may comprise single-chain antibodies. These (inhibiting) antibodies may be linked (e.g. by recombinant techniques) to proteins and/or peptides which provide for their transduction through (cellular) membranes. Corresponding methods are known in the art; see, e.g., Ho (2001), Canc. Res. 61, 474-477; Schwarze (1999), Science 285, 1569-1572; Krautwald (2004), J. Biol. Chem. 279, 44005-44011 or Sujioka (2003), Oncogene 22, 8432-8440.

As documented below, in particular inhibiting or interfering nucleic acid molecules, e.g., antisense siRNA or RNAi may successfully be employed in context of this invention and may be used to modify the SLY-1 activity or function.

Also envisaged are inhibitors/antagonists/negative regulators of SLY-1 function or activity are truncated and/or mutated SLY-1 molecules, which interfere with or are capable of interfering with the complex formation of SLY-1 with its binding partners.

(Small) binding molecules to be employed as inhibitors/ antagonists/ negative regulators of SLY-1 function, activity or expression may comprise natural as well as synthetic compounds. The term "compound" in context of this invention comprises single substances or a plurality of substances. Said compound/binding molecules may be comprised in, for example, samples, e.g., cell extracts from, e.g., plants, animals or microorganisms. Furthermore, said compound (s) may be known in the art but hitherto not known to be capable of (negatively) influencing the activity of SLY-1 or not known to be capable of influencing the expression of the nucleic acid molecule encoding for SLY-1 adaptor proteins, respectively. The plurality of compounds may be, e.g., added to a sample in vitro, to the culture medium or injected into the cell.

If a sample (collection of compounds) containing (a) compound(s) is identified in the art as a specific inhibitory binding molecule of SLY-1, then it is either possible to isolate the compound from the original sample identified as containing the compound in question or one can further subdivide the original sample, for example, if it consists of a plurality of different compounds, so as to reduce the number of different substances per sample and repeat the method with the subdivisions of the original sample. It can then be determined whether said sample or compound displays the desired properties, i.e. the inhibition of SLY-1, by methods known in the art. Depending on the complexity of the samples, the steps described above can be performed several times, preferably until the sample identified according to the screening method only comprises a limited number of or only one substance(s). Preferably said sample comprises substances of similar chemical and/or physical properties, and most preferably said substances are identical.

Binding molecules/inhibitory molecules for SLY-1 may be deduced by methods in the art. Such methods comprise, e.g., but are not limited to methods, where a collection of substances is tested for interaction with SLY-1 adaptor protein or with (a) fragment(s) thereof and where substances which test positive for interaction in a corresponding readout system are further tested in vivo, in vitro or in silico for their inhibitory effects on SLY-1 expression or function.

Said "test for SLY-1 interaction" of the above described method may be carried out by specific immunological, molecular biological and/or biochemical assays which are well known in the art and which comprise, e.g., homogenous and heterogenous assays as described herein below.

In a preferred embodiment, the invention provides for the pharmaceutical composition, use or method as defined herein, wherein said anti-SLY-1-antisense molecule comprises a nucleic acid molecule which is the complementary strand of a reversed complementary strand of the coding region of SLY-1. Most preferably, the anti-SLY-1-antisense molecule to be employed in accordance with this invention is selected from the group consisting of an antisense molecule as shown in any one of SEQ ID NOS: 7 to 16. Further details to antisense molecules are given below.

The SLY-1 molecule to be inhibited in accordance with this invention is preferably a human SLY-1 molecule. However, since also other animals are to be treated, in accordance wit this invention, the SLY-1 molecule to be inhibited is preferably selected from the group consisting of

(a) a SLY-1 molecule encoded by a nucleic acid molecule comprising a nucleic acid molecule as shown in any one of SEQ ID NO: 1, SEQ ID NO: 3 or SEQ ID NO: 5;

(b) a SLY-1 molecule encoded by a DNA molecule coding for a polypeptide having an amino acid sequence as shown in SEQ ID NO: 2; SEQ ID NO: 4 or SEQ ID NO: 6;

(c) a SLY-1 molecule which is encoded by a DNA sequence that hybridizes to the complementary strand of the nucleic acid molecule as defined in (a) or the DNA molecule as defined in (b) and which encodes a polypeptide that is specifically expressed in lymphocytes;

(d) a SLY-1 molecule which comprises an amino acid sequence as shown in SEQ ID NO: 2, 4 or 6;

(e) a SLY-1 molecule which comprises an amino acid sequence which is at least 90% identical to the full-length amino acid sequence as shown in SEQ ID NO: 2, 4 or 6; and

(f) a SLY-1 molecule which is encoded by a DNA sequence which is degenerate to a DNA sequence/nucleic acid molecule as defined in (a) to (c).

The term "specifically expressed in lymphocytes" means that the SLY-1 molecule is specifically or at least most preferentially expressed in B-lymphocytes and/or T-lymphocytes. In contrast to other molecules, like SASH or SLY-2, is the expression of SLY-1 molecules restricted to the expression in lymphocytes; see also Beer (2003) loc. cit.

In accordance with the present invention, the term "nucleic acid sequence" means the sequence of bases comprising purine- and pyrimidine bases which are comprised by nucleic acid molecules, whereby said bases represent the primary structure of a nucleic acid molecule. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art.

When used herein, the term "polypeptide" means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The terms polypeptide and protein are often used interchangeably herein. The term polypeptide also refers to, and does not exclude, modifications of the polypeptide, e.g., glycosylation, acetylation, phosphorylation and the like. Such modifications are well described in basic texts and in more detailed monographs, as well as in a voluminous research literature.

In order to determine whether a nucleic acid sequence has a certain degree of identity to the nucleic acid sequence encoding **SLY-1** adaptor protein, the skilled person can use means and methods well-known in the art, e.g., alignments, either manually or by using computer programs such as those mentioned further down below in connection with the definition of the term "hybridization" and degrees of homology.

For example, BLAST2.0, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments. BLAST produces alignments of both nucleotide and amino acid sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cutoff score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\text{sequence identity} \times \%\text{ maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules.

The present invention also relates to nucleic acid molecules which hybridize to one of the above described nucleic acid molecules and which has CHIP/CHN-1 activity.

The term "hybridizes" as used in accordance with the present invention may relate to hybridizations under stringent or non-stringent conditions. If not further specified, the conditions are preferably non-stringent. Said hybridization conditions may be established according to conventional protocols described, for example, in Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as 0.1xSSC, 0.1% SDS at 65°C. Non-stringent hybridization conditions for the detection of homologous or not exactly complementary sequences may be set at 6xSSC, 1% SDS at 65°C. As is well known, the length of the probe and the composition of the nucleic acid to be determined constitute further parameters of the hybridization conditions. Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Hybridizing nucleic acid molecules also comprise fragments of the above described molecules. Such fragments may represent nucleic acid sequences having a length of at least 12 nucleotides, preferably at least 15, more preferably at least 18, more preferably of at least 21 nucleotides, more preferably at least 30 nucleotides, even more preferably at least 40 nucleotides and most preferably at least 60 nucleotides. Furthermore, nucleic acid molecules which hybridize with any of the aforementioned nucleic acid molecules also include complementary fragments, derivatives and allelic variants of these molecules. Additionally, a hybridization complex refers to a complex between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an antiparallel configuration. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed).

The terms complementary or complementarity refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between single-stranded molecules. The degree of complementartity between nucleic acid strands has significant effects on the efficiency and strength of hybridization between nucleic acid strands. This is of particular importance in amplification reactions, which depend upon binding between nucleic acids strands.

The term "hybridizing sequences" preferably refers to sequences which display a sequence identity of at least 85%, preferably at least 90%, more particularly preferred at least 95%, even more particularly preferred at least 97% or 98% and most preferably at least 99% identity with a nucleic acid sequence as described above encoding SLY-1. Moreover, the term "hybridizing sequences" preferably refers to sequences encoding SLY-1 of at least 90%, preferably at least 95%, particularly preferred at least 97% and most preferably at least 99% identity with an amino acid sequence of SLY-1 as described herein above and/or as shown in SEQ ID NOS: 2, 4 or 6.

In accordance with the present invention, the term "identical" or "percent identity" in the context of two or more nucleic acid or amino acid sequences, refers to two or more sequences or subsequences that are the same, or that have a specified percentage of amino acid residues or nucleotides that are the same, when compared and aligned for maximum correspondence over a window of comparison, or over a designated region as measured using a sequence comparison algorithm as known in the art, or by manual alignment and visual inspection. Sequences having, for example, 90% to 95% or greater sequence identity are considered to be substantially identical. Such a definition also applies to the complement of a test sequence. Preferably the described identity exists over a region that is at least about 15 to 25 amino acids or 45 to 75 nucleotides in length, more preferably, over a region that is about 50 to 100 amino acids or 150 to 300 nucleotides in length and said identity relates to either the amino-terminal region of the proteus or 5'-end of the nucleic acid molecule; see also Figure 1 which documents that SLY-1 molecules are not highly related to SASH or SLY-2 in the their amino terminal region or the region between "Box 1" and the "SH3 domain". Accordingly, SLY-1 molecules in accordance with this invention show a high (>90%) sequence identity to the sequences defined as the first 50 amino acid residues of SLY-1 (as shown in Figure 1 or in any of the sequence shown in SEQ ID Nos: 2, 4, or 6. Also comprised are SLY-1 molecules which comprises a high sequence identity (>90%, preferably >95%) to the sequence defined in Figure 1 between "Box 1" and the "SH3 domain". Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul Nucl. Acids Res. 25 (1977), 3389-3402). The BLASTN program for nucleic acid sequences uses as defaults a word length (W) of 11, an expectation (E) of 10, M=5, N=4, and a comparison of both strands. For amino acid sequences, the BLASTP program uses as defaults a wordlength (W) of 3, and an expectation (E) of 10. The BLOSUM62 scoring matrix (Henikoff Proc. Natl. Acad. Sci., USA, 89, (1989), 10915) uses alignments (B) of 50, expectation (E) of 10, M=5, N=4, and a comparison of both strands.

Moreover, the present invention uses and relates to nucleic acid molecules encoding SLY-1 the sequence of which is degenerate in comparison with the sequence of an above-described hybridizing molecule. When used in accordance with the present invention the term "being degenerate as a result of the genetic code" means that due to the redundancy of the genetic code different nucleotide sequences code for the same amino acid.

The nucleic acid molecule encoding SLY-1 may be any type of nucleic acid, e.g. DNA, RNA or PNA (peptide nucleic acid). A peptide nucleic acid (PNA) is a polyamide type of DNA analog and the monomeric units for adenine, guanine, thymine and cytosine are available commercially (Perceptive Biosystems). Certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (T.sub.m) by 8°-20° C, vs. 4°-16° C for the DNA/DNA 15-mer duplex. Also, the absence of charge groups in PNA means that hybridization can be done at low ionic strengths and reduce possible interference by salt during the analysis. The DNA may, for example, be cDNA. In a preferred embodiment it is a genomic DNA. The RNA may be, e.g., mRNA. The nucleic acid molecule may be natural, synthetic or semisynthetic or it may be a derivative, such as peptide nucleic acid (Nielsen, Science 254 (1991), 1497-1500) or phosphorothioates. Furthermore, the nucleic acid molecule may be a recombinantly produced chimeric nucleic acid molecule comprising any of the aforementioned nucleic acid molecules either alone or in combination.

The term "transplantation" as employed herein relates preferably to an autologous, an allogenic, a homeogenic, a syngenic or a xenogenic transplantation and also relates to homeotransplants. These transplantations are well known in the art and relates not only to the transplantation of cells, but also to the transplantation of tissues and organs, as documented in the appended examples. The transplantation of cells also comprises the transplantation of stem cells.

The term "homeotransplantation" as employed herein relates to a graft of tissue obtained from the body of another animal of the same species but with genotype differing from that of the recipient and/or a tissue graft from a donor of one genotype to a hosts of another genotype, host and donor being members of the same species. Host and donor are called in this respect "allogenic". The allogenic transplantation is provided in the appended examples.

Also comprised in the term "transplant" are "syngrafts" or "isografts", i.e. transplants from one individual to a genetically identical individual of the same species, e.g., genetically identical twins/siblings. In non-human animals these "syngrafts" may also be carried out on transgenic animals.

The term "xenogenic graft/transplantation"/"xenotransplantation" relates to transplantations wherein the graft of tissue (or the cells to be transplanted) are obtained from an animal of one species (e.g., pig or cow) other than that of the recipient (e.g. human). These transplantations also comprise "heterotransplantations".

The present invention is in particular useful, in the medical intervention of graft-versus-host disease, a syndrome arising when in particular an allograft, containing immunocompetent cells, mounts an immune response against a host that is unable to reject it because the host is immunologically immature or immunologically compromised or suppressed (e.g. by radiations or drugs).

The present invention also provides for a method for screening inhibitors or negative regulators of SLY-1 activity or expression comprising the steps of

(a) contacting a cell or a non-human organism expressing SLY-1 or capable of expressing SLY-1 with a compound to be tested;
(b) determining antigen-receptor signal transductions in the presence of said compound to be tested when compared to a cell not contacted with said compound; and
(c) identifying the compound which inhibits SLY-1 activity.

The person skilled in the art is readily position to deduce/determine the antigen receptor signal transduction in cells and/or organisms. One corresponding read-out is the phosphorylation status of SLY-1 adaptor protein itself. It may, inter alia, be deduced whether, upon a corresponding stimulus and in presence or absence of a test compared a Serine-residue being identical to Ser27 or being homologous to Ser27 of SEQ ID NO: 2 is phosphorylated or not. Further screening assays for SLY-1 inhibitors/negative regulators comprise the proliferation of T- and B-cells or the cytokine production in particular the production of IL-2 (interleukin-2) or γ-interferon. Antigen-receptor-signal transduction may, inter alia, be assayed by the use of anti-CD3 antibodies and/or the use of anti-IgM antibodies. The present invention, accordingly provides for specific assays where a SLY-1 inhibitor/antagonist or compounds being suspected to be such an inhibitor or antagonist may be tested.

A SLY-1 inhibitor is, .e.g., capable to inhibit proliferation of lymphocytes which are SLY-1 positive, i.e. lymphocytes/cells that express SLY-1. As control-cells, SLY-1 negative cells (e.g. cells derived from the non-human transgenic animal as described herein and used in the appended examples) may be employed. In SLY-1 negative cells, in particular lymphocytes, a SLY-1 inhibitor does either not alter the proliferation or even leads to an enhanced proliferation.

Furthermore, a screening assay for SLY-1 inhibitors may comprise the measurement of cytokine production in particular in T-cells. Most preferably, the production of IL-2 and/or of interferon-γ is measured. A SLY-1 inhibitor/antagonist leads thereby to a down regulation of said cytokines, in particular of IL-2 and γ-interferon. Again, as specific test, SLY-1 positive cells can be compared to SLY-1 negative cells (derived from the non-human SLY-/SLY- test animal as described in the appended examples). Due to the high sequence homology between mouse SLY-1 and human SLY-1, data and information obtained with mouse cells (in particular SLY-1 positive, wildtype cells in comparison with SLY-1 negative, transgenic cells) are also of high relevance for human cells and the human organism.

The person skilled in the art can easily employ the compounds described herein and the methods of this invention in order to elucidate the inhibitory effects and/or characteristics of a test compound to be identified and/or characterized in accordance with any of the methods described herein and can deduce which is an inhibitor of SLY-1 function, activity or expression.

The term "test compound" or "compound to be tested" refers to a molecule or substance or compound or composition or agent or any combination thereof to be tested by one or more screening method(s) of the invention as a putative inhibitor of SLY-1 function, activity or expression. A test compound can be any chemical, such as an inorganic chemical, an organic chemical, a protein, a peptide, a carbohydrate, a lipid, or a combination thereof or any of the compounds, compositions or agents described herein. It is to be understood that the term "test compound" when used in the context of the present invention is interchangeable with the terms "test molecule", "test substance", "potential candidate", "candidate" or the terms mentioned hereinabove.

Accordingly, small peptides or peptide-like molecules as described hereinabove are envisaged to be used in the screening methods for inhibitor(s) of SLY-1, function, activity or expression. Such small peptides or peptide-like molecules bind to and occupy the active site of a protein thereby making the catalytic site inaccessible to substrate such that normal biological activity is prevented. One of these biological activities may be the phosphorylation status of SLY-1 itself. Moreover, any biological or chemical composition(s) or substance(s) may be envisaged as SLY-1. The inhibitory function of the inhibitor can be measured by methods known in the art and by methods described herein. Such methods comprise interaction assays, like immunoprecipitation assays, ELISAs, RIAs as well as specific assays, like specific phosphorylation assay. In the context of the present application it is envisaged that cells expressing SLY-1 as described herein are used in the screening assays. It is also envisaged that elements of the pathway of antigen-receptor signal transduction are analyzed. Corresponding assays comprise, but are not limited to, the analysis of activation (phosphorylation) of signaling molecules (e.g. MAP-kinases, Y-kinases), the activation of transcription factors (e.g. NF-KB, NFAT, AP-1) or the formation of F-actin.

Also preferred potential candidate molecules or candidate mixtures of molecules to be used when contacting a cell expressing a SLY-1 or an element of the antigen-receptor signal transduction pathway defined and described herein may be, inter alia, substances, compounds or compositions which are of chemical or biological origin, which are naturally occurring and/or which are synthetically, recombinantly and/or chemically produced. Thus, candidate molecules may be proteins, protein-fragments, peptides, amino acids and/or derivatives thereof or other compounds as defined herein, which bind to and/or interact with elements of the antigen-receptor signal transduction pathway. Synthetic compound libraries are commercially available from Maybridge Chemical Co. (Trevillet, Cornwall, UK), Comgenex (Princeton, N.J.), Brandon Associates (Merrimack, N.H.), and Microsource (New Milford, Conn.). A rare chemical library is available from Aldrich (Milwaukee, Wis.). Alternatively, libraries of natural compounds in the form of bacterial, fungal, plant and animal extracts are available from e.g. Pan Laboratories (Bothell, Wash.) or MycoSearch (N.C.), or are readily producible. Additionally, natural and synthetically produced libraries and compounds are readily modified through conventional chemical, physical, and biochemical means.

In addition, the generation of chemical libraries is well known in the art. For example, combinatorial chemistry is used to generate a library of compounds to be screened in the assays described herein. A combinatorial chemical library is a collection of diverse chemical compounds generated by either chemical synthesis or biological synthesis by combining a number of chemical "building block" reagents. For example, a linear combinatorial chemical library such as a polypeptide library is formed by combining amino acids in every possible combination to yield peptides of a given length. Millions of chemical compounds can theoretically be synthesized through such combinatorial mixings of chemical building blocks. For example, one commentator observed that the systematic, combinatorial mixing of 100 interchangeable chemical building blocks results in the theoretical synthesis of 100 million tetrameric compounds or 10 billion pentameric compounds. (Gallop, Journal of Medicinal Chemistry, Vol. 37, No. 9,1233-1250 (1994)). Other chemical libraries known to those in the art may also be used, including natural product libraries. Once generated, combinatorial libraries are screened for compounds that possess desirable biological properties. For example, compounds which may be useful as drugs or to develop drugs would likely have the ability to bind to the target protein identified, expressed and purified as described herein.

In the context of the present invention, libraries of compounds are screened to identify compounds that function as inhibitors of the SLY-1 adaptor proteins. First, a library of small molecules is generated using methods of combinatorial library formation well known in the art. U. S. Patent NOs. 5,463,564 and 5,574,656 are two such teachings. Then the library compounds are screened to identify those compounds that possess desired structural and functional properties. U. S. Patent No. 5,684, 711, discusses a method for screening libraries. To illustrate the screening process, the target cell or gene product and chemical compounds of the library are combined and permitted to interact with one another. A labeled substrate is added to the incubation. The label on the substrate is such that a detectable signal is emitted from metabolized substrate molecules. The emission of this signal permits one to measure the effect of the combinatorial library compounds on the enzymatic activity of target enzymes by comparing it to the signal emitted in the absence of combinatorial library compounds. The characteristics of each library compound are encoded so that compounds demonstrating activity against the cell/enzyme can be analyzed and features common to the various compounds identified can be isolated and combined into future iterations of libraries. Once a library of compounds is screened, subsequent libraries are generated using those chemical building blocks that possess the features shown in the first round of screen to have activity against the target cell/enzyme. Using this method, subsequent iterations of candidate compounds will possess more and more of those structural and functional features required to inhibit the function of the target cell/enzyme, until a group of (enzyme)inhibitors with high specificity for the enzyme can be found. These compounds can then be further tested for their safety and efficacy as antibiotics for use in animals, such as mammals. It will be readily appreciated that this particular screening methodology is exemplary only. Other methods are well known to those skilled in the art. For example, a wide variety of screening techniques are known for a large number of naturally-occurring targets when the biochemical function of the target protein is known. For example, some techniques involve the generation and use of small peptides to probe and analyze target proteins both biochemically and genetically in order to identify and develop drug leads. Such techniques include the methods described in PCT publications No. WO 99/35494, WO 98/19162, WO 99/54728.

Preferably, candidate agents to be tested in the encompass numerous chemical classes, though typically they are organic molecules, preferably small organic compounds having a molecular weight of more than 50 and less than about 2,500 Daltons, preferably less than about 750, more preferably less than about 350 daltons. Candidate agents may also comprise functional groups necessary for structural interaction with proteins, particularly hydrogen bonding, and typically include at least an amine, carbonyl, hydroxyl or carboxyl group, preferably at least two of the functional chemical groups. The candidate agents often comprise carbocyclic or heterocyclic structures and/or aromatic or polyaromatic structures substituted with one or more of the above functional groups.

Exemplary classes of candidate agents may include heterocycles, peptides, saccharides, steroids, and the like. The compounds may be modified to enhance efficacy, stability, pharmaceutical compatibility, and the like. Structural identification of an agent may be used to identify, generate, or screen additional agents. For example, where peptide agents are identified, they may be modified in a variety of ways to enhance their stability, such as using an unnatural amino acid, such as a D-amino acid, particularly D-alanine, by functionalizing the amino or carboxylic terminus, e.g. for the amino group, acylation or alkylation, and for the carboxyl group, esterification or amidification, or the like. Other methods of stabilization may include encapsulation, for example, in liposomes, etc. As mentioned above, candidate agents are also found among biomolecules including peptides, amino acids, saccharides, fatty acids, steroids, purines, pyrimidines, nucleic acids and derivatives, structural analogs or combinations thereof. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

The non-human organism to be used in said screening method is preferably selected from the group consisting of C. elegans, yeast, drosophila, zebrafish, mouse, rat, guinea pig, dog, cat.

The screening method provided herein may also comprise the determination of a given molecule to be suspected inhibit SLY-1 protein (or the SLY-1 expression product defined herein) is capable to "interact with" said adaptor protein. Corresponding "interactions" are tested in interaction assays.

Said interaction assays employing read-out systems are well known in the art and comprise, inter alia, two hybrid screenings (as, described, inter alia, in EP-0 963 376, WO 98/25947, WO 00/02911), GST-pull-down columns, co-precipitation assays from cell extracts as described, inter alia, in Kasus-Jacobi, Oncogene 19 (2000), 2052-2059, "interaction-trap" systems (as described, inter alia, in US 6,004,746) expression cloning (e.g. lamda gtII), phage display (as described, inter alia, in US 5,541,109), in vitro binding assays and the like. Further interaction assay methods and corresponding read out systems are, inter alia, described in US 5,525,490, WO 99/51741, WO 00/17221, WO 00/14271, WO 00/05410 or Yeast Four hybrid assays as described in Sandrok & Egly, JBC 276 (2001), 35328-35333.

Said interaction assays for SLY-1 also comprise assays for FRET-assays, TR-FRETs (in "A homogenius time resolved fluorescence method for drug discovery" in: High throughput screening: the discovery of bioactive substances. Kolb, (1997) J.Devlin. NY, Marcel Dekker 345-360) or commercially available assays, like "Amplified Luminescent Proximity Homogenous Assay", BioSignal Packard. Furthermore, the yeast-2-hybrid (Y2H) system may be employed to elucidate further particular and specific interaction, association partners of SLY-1. Said interaction/association partners are further screened for their inhibitory effects, in particular also their effect on ubiquitylation.

Similarly, interacting molecules (for example) (poly)peptides may be deduced by cell-based techniques well known in the art. These assays comprise, inter alia, the expression of reporter gene constructs or "knock-in" assays, as described, for, e.g., the identification of drugs/small compounds influencing the (gene) expression of SLY-1. Said "knock-in" assays may comprise "knock-in" of SLY-1 (or (a) fragment(s) thereof) in tissue culture cells, as well as in (transgenic) animals. Examples for successful "knock-ins" are known in the art (see, inter alia, Tanaka, J. Neurobiol. 41 (1999), 524-539 or Monroe, Immunity 11 (1999), 201-212). Furthermore, biochemical assays may be employed which comprise, but are not limited to, binding of the SLY-1 (or (a) fragment(s) thereof) to other molecules/(poly)peptides, peptides or binding of the SLY-1 (or (a) fragment(s) thereof) to itself (themselves) (dimerizations, oligomerizations, multimerizations) and assaying said interactions by, inter alia, scintillation proximity assay (SPA) or homogenous time-resolved fluorescence assay (HTRFA).

Said "testing of interaction" may also comprise the measurement of a complex formation. The measurement of a complex formation is well known in the art and comprises, inter alia, heterogeneous and homogeneous assays. Homogeneous assays comprise assays wherein the binding partners remain in solution and comprise assays, like agglutination assays. Heterogeneous assays comprise assays like, inter alia, immuno assays, for example, ELISAs, RIAs, IRMAs, FIAs, CLIAs or ECLs.

The "interaction assay" may lead to specific inhibitors of SLY-1 protein (or the SLY-1 expression product) which may be further tested in specific interaction assays. Accordingly, SLY-1 inhibitors in the sense of the present invention are also compounds which influence the expression of the SLY-1 encoding nucleic acid molecules, e.g. siRNAs, antisense molecules (as, inter alia, given in appended SEQ ID NOS: 7 to 16), RNAis, aptamers and the like.

As discussed below the interaction of the inhibiting molecules of SLY-1 mRNA and SLY-1 protein or fragments thereof may also be tested by molecular biological methods, like two-, three- or four-hybrid-assays, RNA protection assays, Northern blots, Western blots, micro-, macro- and Protein- or antibody arrays, dot blot assays, in situ hybridization and immunohistochemistry, quantitative PCR, coprecipitation, far western blotting, phage based expression cloning, surface plasmon resonance measurements, yeast one hybrid screening, DNAse I, footprint analysis, mobility shift DNA-binding assays, gel filtration chromatography, affinity chromatography, immunoprecipitation, one- or two dimensional gel electrophoresis, aptamer technologies, as well as high throughput synthesis and screening methods.

The compounds identified and/or obtained according to the above described method(s), in particular inhibitors of SLY-1 or (a) fragment(s) thereof, are expected to be very beneficial as agents in pharmaceutical settings disclosed herein and to be used for medical purposes, in particular, in transplantation medicine.

As mentioned above, compounds which may function as specific inhibition of SLY-1 also comprise (small) organic compounds, like compounds which can be used in accordance with the present invention include, inter alia, peptides, proteins, nucleic acids including cDNA expression libraries, small organic compounds, ligands, PNAs and the like. Said compounds can also be functional derivatives or analogues. Methods for the preparation of chemical derivatives and analogues are well known to those skilled in the art and are described in, for example, Beilstein, "Handbook of Organic Chemistry", Springer Edition New York, or in "Organic Synthesis", Wiley, New York. Furthermore, said derivatives and analogues can be tested for their effects, i.e. their inhibitory effects of SLY-1 according to methods known in the art. Furthermore, peptidomimetics and/or computer aided design of appropriate inhibitors of SLY-1 can be used. Appropriate computer systems for the computer aided design of, e.g., proteins and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known compounds, substances or molecules. Appropriate compounds can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of inhibitors of SLY-1 can be used for the design of (peptidomimetic) inhibitors of SLY-1 (Rose, Biochemistry 35 (1996), 12933-12944; Rutenber, Bioorg. Med. Chem. 4 (1996), 1545-1558).

As discussed herein above, the inhibitor of SLY-1 expression or function may also comprise an aptamer.
In the context of the present invention, the term "aptamer" comprises nucleic acids such as RNA, ssDNA (ss = single stranded), modified RNA, modified ssDNA or PNAs which bind a plurality of target sequences having a high specificity and affinity. Aptamers are well known in the art and, inter alia, described in Famulok, Curr. Op. Chem. Biol. 2 (1998), 320-327. The preparation of aptamers is well known in the art and may involve, inter alia, the use of combinatorial RNA libraries to identify binding sites (Gold, Ann. Rev. Biochem. 64 (1995), 763-797).

Accordingly, aptamers are oligonucleotides derived from an in vitro evolution process called SELEX (systematic evolution of ligands by exponential enrichment). Pools of randomized RNA or single stranded DNA sequences are selected against certain targets. The sequences of tighter binding with the targets are isolated and amplified. The selection is repeated using the enriched pool derived from the first round selection. Several rounds of this process lead to winning sequences that are called 'aptamers' or 'ligands'. Aptamers have been evolved to bind proteins which are associated with a number of disease states. Using this method, many powerful antagonists of such proteins can be found. In order for these antagonists to work in animal models of disease and in humans, it is normally necessary to modify the aptamers. First of all, sugar modifications of nucleoside triphosphates are necessary to render the resulting aptamers resistant to nucleases found in serum. Changing the 2'OH groups of ribose to 2'F or 2'NH2 groups yields aptamers which are long lived in blood. The relatively low molecular weight of aptamers (8000-12000) leads to rapid clearance from the blood. Aptamers can be kept in the circulation from hours to days by conjugating them to higher molecular weight vehicles. When modified, conjugated aptamers are injected into animals, they inhibit physiological functions known to be associated with their target proteins. Aptamers may be applied systemically in animals and humans to treat organ specific diseases (Ostendorf (2001) J. Am Soc. Neph. 12, 909-918). The first aptamer that has proceeded to phase I clinical studies is NX-1838, an injectable angiogenesis inhibitor that can be potentially used to treat macular degeneration- induced blindness. (Sun (2000) Curr. Op. Mol Ther. 2, 100-105). Cytoplasmatic expression of aptamers ("intramers") may be used to inhibit intracellular targets (Mayer (2001) PNAS 98, 4961-4965). Said intramers are also envisaged to be employed in context of this invention.

Said (other) receptors to be employed as "inhibitors/antagonists of SLY-1" may, for example, be derived from (an) antibody(ies) against SLY-1 by peptidomimetics. The specificity of the recognition implies that other known proteins, molecules are not bound.

The RNAi-approach is also envisaged in context of this invention for use in the preparation of a pharmaceutical composition for the treatment of muscular diseases disclosed herein.

The term RNA interference (RNAi) describes the use of double-stranded RNA to target specific mRNAs for degradation, thereby silencing their expression. Double-stranded RNA (dsRNA) matching a gene sequence is synthesized in vitro and introduced into a cell. The dsRNA feeds into a natural, but only partially understood process including the highly conserved nuclease dicer (Hutvàgner, 2001; Grishok, 2001), which cleaves dsRNA precursor molecules into short interfering RNAs (siRNAs). The generation and preparation of siRNA(s) as well as the method for inhibiting the expression of a target gene is, inter alia, described in WO 02/055693, Wei (2000) Dev. Biol. 15, 239-255; La Count (2000), Biochem. Paras. 111, 67-76, Baker (2000) Curr. Biol. 10, 1071-1074, Svoboda (2000), Development 127, 4147-4156 or Marie (2000) Curr. Biol. 10, 289-292. These siRNAs built then the sequence specific part of an RNA-induced silencing complex (RISC), a multicomplex nuclease that destroys messenger RNAs homologous to the silencing trigger. One protein-part of the ribonucleoprotein complex has been identified as Argonaute2 (Hammond, 2001). Elbashir (2001) showed that duplexes of 21 nucleotide RNAs may be used in cell culture to interfere with gene expression in mammalian cells.

Methods to deduce and construct siRNAs are in the art and are describedin the appended examples as well as in Elbashir et al., 2002, at the internet web sites of commercial vendors of siRNA, e.g. Xeragon Inc. (www.xeragon.com/siRNA support.html); Dharmacon (www.dharmacon.com); Xeragon Inc. (www.xeragon.com), and Ambion (www.ambion.com), or at the web site of the research group of Tom Tuschl (http://www.rockefeller.edu/labheads/tuschl/ index.html). In addition, programs are available online to deduce siRNAs from a given mRNA sequence (e.g. http://www.ambion.com/techlib/misc/siRNA finder.html or http://katahdin.cshl.org:9331/RNAi/). Uridine residues in the 2-nt 3' overhang can be replaced by 2'deoxythymidine without loss of activity, which significantly reduces costs of RNA synthesis and may also enhance resistance of siRNA duplexes when applied to mammalian cells (Elbashir, 2001). This modification is also incorporated in citing SEQ Ids 44-83 (see below) of the present application. The siRNAs may also be sythesized enzymatically using T7 or other RNA polymerases (Donze, 2002). Short RNA duplexes that mediate effective RNA interference (esiRNA) may also be produced by hydrolysis with Escherichia coli Rnase III (Yang, 2002) Furthermore, expression vectors have been developed to express double stranded siRNAs connected by small hairpin RNA loops in eukaryotic cells (e.g. (Brummelkamp, 2002)).All of these constructs may by developed with the help of the programs named above. In addition, commercially available sequence prediction tools incorporated in sequence analysis programs or sold separately, e.g. the siRNA Design Tool offered by www.oligoEngine.com (Seattle,WA) may be used for siRNA sequence prediction.

Accordingly, the present invention also provides for the use of specific interfering RNAs as inhibitors of SLY-1 expression, activity and/or function. Preferably, said (small) interfering RNAs (siRNAs) comprise at least 10, more preferably at least 12, more preferably at least 14, more preferably at least 16, more preferably at least 18 nucleotides.

As mentioned above, intracellular antibodies are known in the art and can be used to neutralize or modulate the functional activity of SLY-1. This therapeutic approach is based on intracellular expression of recombinant antibody fragments, either Fab or single chain Fv, targeted to the desired cell compartment using appropriate targeting sequences (summarized in Teillaud (1999) Pathol. Biol. 47, 771-775). Further sequences (transduction sequences) are given above.

A further preferred inhibitor, SLY-1 expression and/or function is an antisense molecule. Preferably said anti-SLY-1 antisense molecule comprises a nucleic acid molecule which is the complementary strand of a reversed complementary strand of the coding region of SLY-1. As documented above, preferred antisense molecules are given in SEQ ID NOS: 7 to 16.

Coding regions of SLY-1 are known in the art and comprise, inter alia, SLY-1 GenBank entries for mouse (AJ306422), for rat (XM_229107) or for human (NM_018990). The person skilled in the art may easily deduce the relevant coding region of SLY-1 in these GenBank entries, which may also comprise the entry of genomic DNA as well as mRNA/cDNA.

Furthermore, it is also envisaged that the antisense molecules against SLY-1 expression, activity or function interfere specifically with promoter regions of SLY-1.

It is envisaged that the antisense molecules to be used in accordance with the present invention inhibit the expression or function of SLY-1, in particular of human SLY-1 and interact with SLY-1 as expressed by the coding regions, mRNAs/cDNAs as deposited under the above mentioned GenBank accession numbers as well as with SLY-1 as expressed by isoforms and variants of said SLY-1. Said isoforms or variants may, inter alia, comprise allelic variants or splice variants.

The term "variant" means in this context that the SLY-1 nucleotide sequence and the encoded SLY-1 amino acid sequence, respectively, differs from the distinct sequences available under said GenBank Accession numbers, by mutations, e.g. deletion, additions, substitutions, inversions etc..

Therefore, the antisense-molecule to be employed in accordance with the present invention specifically interacts with/hybridizes to one or more nucleic acid molecules encoding SLY-1. Preferably said nucleic acid molecule is RNA, i.e. pre mRNA or mRNA. The term "specifically interacts with/hybridizes to one or more nucleic acid molecules encoding SLY-1" relates, in context of this invention, to antisense molecules which are capable of interfering with the expression of SLY-1. The person skilled in the art can easily deduce whether an antisense construct specifically interacts with/ hybridizes to SLY-1 encoding sequences. These tests comprise, but are not limited to hybridization assays, RNAse protection assays, Northern Blots, North-western blots, nuclear magnetic resonance and fluorescence binding assys, dot blots, micro- and macroarrays and quantitative PCR. In addition, such a screening may not be restricted to SLY-1 mRNA molecules, but may also include SLY-1 mRNA/protein (RNP) complexes. Furthermore, functional tests as provided in the appended examples are envisaged for testing whether a particular antisense construct is capable of specifically interacting with/hybridizing to the SLY-1 encoding nucleic acid molecules. These functional assays comprise ubiquitylation assays provided in the examples. These functional tests may also include Western blots, immunohistochemistry, immunoprecipitation assay, and bioassays based on, inter alia, GFP reporter gene fusions.

The term "antisense-molecule" as used herein comprises in particular antisense oligonucleotides. Said antisense oligonucleotides may also comprise modified nucleotides as well as modified internucleoside-linkage, as, inter alia, described in US 6,159,697. Most preferably, the antisense oligonucleotides of the present invention comprise at least 8, more preferably at least 10, more preferably at least 12, more preferably at least 14, more preferably at least 16 nucleotides. The deduction as well as the preparation of antisense molecules is very well known in the art. The deduction of antisense molecules is, inter alia, described in Smith, 2000. Usual methods are "gene walking", RnaseH mapping, RNase L mapping (Leaman and Cramer, 1999), combinatorial oligonucleotide arrays on solid support, determination of secondary structure analysis by computational methods (Walton, 2000), aptamer oligonucleotides targeted to structured nucleic acids (aptastruc), thetered oligonucleotide probes, foldback triplex-forming oligonucleotides (FTFOs) (Kandimalla, 1994) and selection of sequences with minimized non-specific binding (Han, 1994).

Preferably, the antisense molecules of the present invention are stabilized against degradation. Such stabilization methods are known in the art and, inter alia, described in US 6,159,697. Further methods described to protect oligonucleotides from degradation include oligonucleotides bridged by linkers (Vorobjev, 2001), minimally modified molecules according to cell nuclease activity (Samani, 2001), 2'O-DMAOE oligonucleotides (Prakash, 2001), 3'5'-Dipeptidyl oligonucleotides (Schwope, 1999), 3'methylene thymidine and 5-methyluridine/cytidine h-phosphonates and phosphonamidites (An, 2001), as well as anionic liposome (De Oliveira, 2000) or ionizable aminolipid (Semple, 2001) encapsulation.

In a preferred embodiment of the invention, the antisense molecule is a nucleic acid molecule which is the complementary strand of a reversed complementary strand of the coding region of SLY-1 is selected from any of SEQ ID NO: 1, 3 and 5. Sequence as depicted in SEQ ID NOs: 1, 3 or 5 represents illustrative coding regions (mRNA) of mouse, human or rat. SEQ ID NOs: 2, 4 or 6 represent translated SLY-1 of mouse, human or rat, respectively. Accordingly, in context of this invention and as stressed herein above, the SLY-1 inhibitors to be employed in the uses described herein, preferably, interact with promoter and/or coding regions of nucleic acid molecules which code for or lead to the expression of SLY-1 molecules as shown in SEQ ID NOs: 2, 4 or 6. It is also envisaged that, e.g., antisense constructs designed and used in accordance with this invention inhibit the expression of functional homologues, variants (for example allelic variants) or isoforms of SLY-1 as shown in SEQ ID NOs: 2, 4 or 6.

In context of this invention, the term "coding region of SLY-1" comprises not only the translated region of SLY-1, but also comprises untranslated regions. Accordingly, the anti-SLY-lantisense molecule to be used and employed in accordance with this invention may be antisense molecules which bind to/interact with mRNA sequences comprising untranslated region.

The invention also relates to a method for preventing, ameliorating and/or treating a transplantation related disorder comprising administering a specific inhibitor of SLY-1 expression or function as defined herein above to a subject in need thereof. Preferably, said subject is a mammal, most preferably said mammal is a human.

A "patient" or "subject" for the purposes of the present invention includes both humans and other animals, particularly mammals, and other organisms. Thus, the methods are applicable to both human therapy and veterinary applications. In the preferred embodiment the patient is a mammal, and in the most preferred embodiment the patient is human.

Most preferably, the pharmaceutical composition to be prepared in accordance with this invention and comprising an SLY-1 expression and/or function inhibitor(s) is to be administered by one or several of the following modes: Administration can be oral, intravenous, intraarterial, intratracheal, intranasal, subcutaneous, intramuscular, intracranial (i.e. intraventricular) or intraspinal (intrathecal), epidermal or transdermal, pulmonary (e.g. inhalation or insufflation of aerosol or powder), by delivery to the oral or rectal mucosa as well as ophthalmic delivery. Preferred in context of this invention is a intramuscular administration.

It is envisaged that the cells, tissues and/or organs to be transplanted are, before or during transplantation to be treated with the SLY-1 inhibitors/antagonists as defined herein.

Dosage and administration regimes for the SLY-1 may be established by the physician. For example, for antisense compounds, like antisense-nucleotides specific dosage regimes have been established. Such regimes comprise a dosage of 1 mg/kg up to 200 $mg/m^2$ and are, inter alia, described in Schreibner (2001), Gastroenterology 120, 1399-1345; Andrews (2001), J. Clin. Oncol. 19, 2189-2200; Blay (2000), Curr. Op. Mol. Ther. 2, 468-472; Cunnigham (2000), Clin. Cancer Res. 6, 1626-1631; Waters (2000), J. Clin. Oncol. 18, 1809-1811 or Yacyshyn (1998), Gastroenterology 114, 1133-1142. It is, for example, envisaged that the SLY-1 described herein, e.g. antisense compounds or RNAi and the like, be administered in single doses of 0.1 to 25 mg/kg/die (for example i.v. over 2 to 8 hours), as single or multiple doses every other day or by continuous infusion(s) of 0.5 to 10 mg/kg/die over 14 to 21 days with 7 day rest. It is of particular note that in certain clinical or medical indications it might be desirable to administer the SLY-1 as disclosed herein in a single dose. Further dosage regimes are envisaged and may easily be established by a physician.

The Figures show:

**<u>Fieure 1:</u> Generation of *Sly1*-deficient mice.**

(a) Amino acid sequence alignment of murine SLY1, SLY2, and SASH1 proteins is shown. Identical residues are shaded and protein domains are indicated by boxes. (b) The organization of part of the murine *Sly1* genomic locus (top), the linearized targeting vector (middle), and the targeted *Sly1* allele (bottom) are shown. The β-galactosidase cDNA and the neomycin resistance cassette were inserted between codon 26 (exon 2) and codon 72 (exon 3) of the *Sly1* gene. The β-galactosidase cDNA contains a polyadenylation signal thereby preventing aberrant splicing of the targeted gene. Exon sequences are represented by filled rectangles, intron sequences by a black line. Restriction sites are R (Eco RI) and the relevant restriction fragments for detection of the wild-type or mutant allele are shown. The location of the probe fragment for Southern blot hybridization is indicated. (c) The absence of SLY1 expression in splenic mRNA of a representative male mouse hemizygous for the mutant *Sly1* allele (lane 1) was verified by RT-PCR with *Sly1*-specific primers. SLY1 expression was detected in splenic mRNA from a wild-type littermate control (lane 2). Expression of mRNA for GAPDH served as control.

**<u>Figure 2:</u> Hypoplasia of lymphoid organs and altered lymphocyte subsets in *Sly1*-deficient mice.**

**(a)** Total cell numbers were determined from bone marrow (BM), thymus (THY), spleen (SPL), mesenteric (MLN) and peripheral (PLN) lymph nodes, Peyer's patches (PP), and in peritoneal lavage (PEC) of wild-type littermates (gray bars) and *Sly1*-deficient mice (filled bars) with 18 mice analyzed in each group. **(b)** Numbers of macroscopically visible Peyer's patches were counted. Each symbol represents an individual mouse. WT, wild-type littermate controls; KO, *Sly1*-deficient mice. (c) The proportion of CD4 and CD8 T cells (top panel; gated on $CD3^+$ cells) and marginal zone B cells (bottom panel; gated on $IgM^+$ cells) in spleens of wildtype and *Sly1*-deficient mice were determined by flow cytometry. The percentage of lymphocyte subsets is given in each gate. The histograms are representative of at least 6 independent mice per group. *, p< 0.05; ***, p < 0.001 (Student's *t* test). (c)

**<u>Figure 3:</u> Impaired B cell proliferation and reduced basal immunoglobulin levels in *Sly1*-deficient mice.**

**(a)** Splenocytes obtained from wildtype (gray bars) and *Sly1*-deficient mice (filled bars) were stimulated with anti-IgM at the indicated concentrations. IL-4 (10 ng/ml) or LPS (40 ng/ml) were added as indicated. $^3$[H]-Thymidine incorporation was measured after 3 d in all experiments. The data are representative of 12 independent mice per group. **(b)** Basal levels of various immunoglobulin isotypes were determined in serum of untreated mice. The data are derived from 17-19 independent mice per group. *, p< 0.05; ***, p < 0.001 (Student's *t* test).

**<u>Figure 4:</u> Impaired humoral immune responses in *Sly1*-deficient mice.**

(**a**) For analysis of T-independent antibody responses, the time course of TNP-specific IgM and IgG3 antibody levels was determined in serum of wildtype (triangles) and *Sly1*-deficient mice (circles) immunized with TNP-Ficoll. Data are derived from 7-8 independent mice per group. (**b**) T-dependent production of TNP-specific antibody isotypes was measured after immunization of mice with TNP-CGG adsorbed to alum (n = 4 per group for d4 and d7; n = 8 per group for d0, d14, d21). *, p< 0.05; **, p < 0.01; ***, p < 0.001 (Student's *t* test).

**<u>Figure 5</u>: Impaired antigen receptor-triggered cytokine production of *Sly1*-deficient T cells.**

(**a**) Splenocytes from wildtype (gray bars) or Slyl-deficient mice (filled bars) were stimulated with CD3 (2 μg/ml) or CD3 (0.4 μg/ml) and CD28 (2.5 μg/ml) antibodies and cytokine production was measured after 18h. Data are derived from 4 mice per group. (**b**) Splenocytes were stimulated with the indicated concentrations of PMA and inonomycin and IL-2 release was determined after 18h. Data are derived from 4-6 mice per group. *, p< 0.05; **, p < 0.01 (Student's *t* test).

**Figure 6: Reduced T cell proliferation in the absence of SLY1 is rescued by exogenous IL-2.**

(**a**) Splenocytes from wildtype (gray bars) or *Sly1*-deficient mice (filled bars) were stimulated by the addition of antibodies against CD3. CD28 antibodies (2.5 $\mu$g/ml) or IL-2 (10 ng/ml) were added as indicated. Data are derived from 12 independent mice per group. *, p< 0.05; **, p < 0.01 (Student's t test). **(b**) Splenocytes were left unstimulated or incubated with CD3 antibodies (2 $\mu$g/ml) for 16h. Cell surface expression of CD25 and CD69 on splenic CD4 and CD8 T cells from wildtype (solid lines) and *Sly*1-deficient mice (dotted lines) was determined by flow cytometry. Histograms are representative of 5 independent mice per group.

**Figure 7: Distinct effects of *Sly1* deficiency on allogeneic mixed lymphocyte reactions and cytotoxic T cell activity.**
Total splenocytes (**a**) or splenocytes depleted from CD8 (**b**) or CD4 (**c**) T cells were prepared from Slyl-deficient mice (KO) or wild-type littermates (WT) and stimulated with irradiated BALB/c splenocytes or autologous cells. Data are derived from 5 independent mice per group. *, p< 0.05; **, p < 0.01 (Student's *t* test). (**d**) Mice were immunized with OVA in the presence or absence of CpG-DNA (CpG) as an adjuvant. Draining lymph node cells were harvested 4 days later and cultured *in vitro* with rIL-2 for an additional 4 days. Cytotoxic T cell activity was assayed using EL4 target cells pulsed with the SIINFEKL peptide. Data are derived from 4 independent experiments.
**Figure 8: Improved cardiac allograft acceptance in *Sly1* -deficient mice.**
Fully allogeneic H-$2^d$ **(a)** or semi-identical H-$2^{bxd}$ **(b)** cardiac allograft were transplanted into wildtype or *Sly1*-deficient mice. Allograft survival was monitored for the indicated time periods. As a control, wildtype mice received syngeneic H-$2^b$ grafts.

The examples illustrate the invention.

**Example 1: Experimental Procedures**

**Reagents**

Antibodies against the surface markers CD3ε (145-2C11), CD4 (L3T4), CD8 (53-6.7), CD21 (7G6), CD23 (B3B4), CD25 (PC61), CD28 (37.51), CD69 (H1.2F3), B220 (RA3-6B2), IgD (11-26), and IgM (R60-60.2) were from BD Pharmingen. Anti-IgM F(ab')2 was from Jackson ImmunoResearch, rIL-2 and rIL-4 were from R&D Systems, and LPS was from Sigma. Concentrations of IL-2, IL-4, IL-10, TNF and IFN-γ in culture supernatants were measured using ELISA kits from R&D Systems. TNP-CGG, TNP-Ficoll and TNP-BSA were from Biosearch Technologies, and Alum was obtained from Serva. PMA and ionomycin were purchased from Sigma.

**Gene targeting and mice**

E14.1 embryonic stem (ES) cells from 129/Ola mice were grown in DMEM medium (GIBCO BRL) supplemented with L-glutamine (2 mM; Seromed), leukemia inhibitory factor, penicillin/streptomycin (100$\mu$g/ml; Seromed), 2-mercaptoethanol (0,05 mM; GIBCO BRL), and 15% heat-inactivated FCS (Boehringer).
A murine genomic ES-129 BAC library (Genome Systems Inc.) was screened by hybridization with a 205 bp murine *Slyl* cDNA fragment as a probe. The resulting BAC clone was mapped by Southern blot hybridization using murine *Slyl* cDNA fragments. BAC fragments were cloned into pBluescript (Stratagene) and fully sequenced. The targeting vector was constructed in pBluescript such that a 600 bp fragment of the genomic *Sly1* locus encoding exon 2 and 3 was replaced by lacZ and a neomycin resistance cassette. A herpes simplex virus thymidine kinase (HSV-TK) cassette was inserted 3 kb upstream of the targeted sequence. E14.1 ES cells were electroporated with the NotI-linearized targeting vector, and the transfected cells subjected to G418 and gancyclovir selection. Homologous recombinated clones were detected by PCR and subsequently confirmed by Southern blot hybridization with the 3' flanking probe indicated in **Fig. 1a** after digestion **of ES** cell DNA with EcoRI. Single integration was verified by probing the Southern blot with the neomycin resistance cassette. Correctly targeted ES cell clones were injected into C57BL/6 blastocysts, which were transferred into pseudopregnant foster mothers. Resulting chimeric mice were backcrossed to C57BIJ6 mice, and germline transmission of the targeted allele was confirmed by Southern blot analysis.
Mice were kept according to national guidelines for animal care in a SPF animal facility. The mice used in these experiments were backcrossed to the C57BL./6 background for 6-8 generations. Wild-type littermates were used as controls. Genotyping for the *Slyl* mutation was performed by PCR with the following primers: 5' GAT CCA TGC CAG CGT TAC CA (WT/ forward); 5' TCG CCT TCT ATC GCC TTC TTG (Neo/for) and 5' AGT CAT AGC TCT CCA TCA GC (reverse).

**Lymphocyte proliferation and cytokine production**

Splenocytes were incubated at $2x10^5$ cells per well in 96 well round-bottom plates with the indicated soluble antibodies or reagents in 200 μl media. For proliferation assays, 1 μCi [$^3$H]-thymidine (Amersham Pharmacia Biotech) was added after 66h of culture and incubated for 16h. Incorporation of [$^3$H]-thymidine was measured with a Matrix™ 96 direct γ counter (Packard Instrument Co.). For analysis of cytokine production, supernatants were harvested after 18h of stimulation and assayed by ELISA (all R&D Systems).

**Allogeneic mixed lymphocyte reaction**

For induction of mixed lymphocyte reactions, allogeneic splenocytes (H-2$^d$) were used as stimulator cells and cocultured with responder cells at concentrations of 2 x $10^6$ cells/ml. The stimulator population was irradiated with 30Gy prior to coculture. The responder cells were total splenocytes (1,2 x $10^6$ cells/ml), CD4-depleted splenocytes (1,5 x $10^6$ cells/ml), or CD8-depleted splenocytes (1,7 x $10^6$ cells/ml). Cell depletion was performed using anti-CD4 or anti-CD8 microbeads according to the manufacturer's instructions (Milenyi Biotec). Depletion efficiency as determined by flow cytometry analysis was >99%. Proliferation was measured after 84h of MLR activation by addition of 1μCi/ well $^3$[H]-thymidine (Amersham Pharmacia Biotec) during the last 10 h of culture.

Cytotoxic lymphocyte assay

Mice were immunized s.c. into the hind footpad with OVA (150 μg/footpad; OVA Grade IV, Sigma) using CpG-DNA 1668 (5 nmol/footpad) as an adjuvant. Draining lymph nodes were removed 4 days later and cells were cultured for an additional 4 days in medium supplemented with 10 U/ml rIL-2, which allows expansion of *in vivo* primed CTL precursors. $^{51}$Cr-release assays were performed with EL4 target cells pulsed with 0.1 μM of the OVA-derived peptide SIINFEKL. Serial dilutions of effector CTL were added to target cells and $^{51}$Cr-release was measure after 4h of coculture. Specific lysis was calculated according to the formula: % specific lysis = [cpm (sample) - cpm (spontaneous release) / cpm (maximal release - cpm (spontaneous release)] X 100. For control, unpulsed EL4 cells were used as targets. Specific lysis of unpulsed EL4 cells was below 2% in all experiments.

**Humoral immune responses**

Mice were immunized i.p. with 5 μg TNP-CGG ($NP_{19}$-CGG) adsorbed to alum or with 100 μg TNP-Ficoll in PBS. Sera were collected before and 4, 7, 14 and 21 days after immunization. Total NP-specific immunoglobulin isotypes (IgM, IgG1, and IgG2a) were detected employing an ELISA with $NP_{14}$-bovine-serum-albumin ($NP_{14}$-BSA)-conjugated plates (10 μg/ml in carbonate-buffer pH9.5). Sera were diluted 1:1000 for TNP-CGG/alum and 1:500 for TNP-Ficoll immunizations, respectively. Murine NP-specific antibodies were detected with alkaline phosphatase-conjugated isotype-specific detection antibodies, which were obtained from Pharmingen (anti-IgGl, and anti-IgG2a) or Jackson ImmunoResearch (anti-IgM).

**Allograft transplantations**

Vascularized cardiac grafts from donor mice were collected in anesthesia by dividing and excising the aorta and pulmonary artery as described (Maier et al., 2001). Donor hearts were placed in Brettschneider's solution (HTK Köhler Chemie, Germany) until grafting. Abdominal aorta and vena cava were prepared in recipient mice. Subsequently, the cardiac grafts were heterotypically transplanted by end-to-side anastomosis of donor aorta and pulmonary artery to the host's aorta and vena cava, respectively. Resumption of cardiac function of donor hearts was verified. Graft function was monitored daily by transabdominal palpation and scored. Rejection was defined as complete cessation of palpable heart beats and confirmed by direct inspection after laparotomy.

**Example 2: Altered lymphoid organogenesis and lymphocyte development in *Sly1*-deficient mice**

The protein sequence analysis depicted in Figure 1a demonstrates that SLY1 exhibits extensive homology to SLY2 (previously termed Nashl, HACS1, or SAMSN1) and SASH1 proteins (Uchida et al., 2001; Claudio et al., 2001; Zeller et al., 2003). SLY1, SLY2, and SASHI show a similar domain organization with a unique N-terminal sequence (box 1) followed by SH3 and SAM domains and a second unique C-terminal sequence (box 2). SASH1 contains unique N- and C-terminal sequence extension as compared to SLY1 and SLY2. Data base searches showed that the respective SH3 and SAM domains of SLY1, SLY2, and SASH1 are substantially more homologous to each other than to SH3 and SAM domains of other proteins suggesting that they define separate subfamilies of these protein modules (data not shown). Interestingly, the second SAM domain of SASH1 that is located at the C-terminus of the protein is less well conserved. Due to their extensive sequence homology and unique domain organization SLY1, SLY2, and SASH1 may therefore form a new family of adaptor proteins.
Although SLY1, SLY2, and SASH1 are all highly expressed in hematopoietic cells, only the expression of SLY1 is restricted to the lymphoid lineage (Beer et al., 2001; Claudio et al., 2001; Re et al., 2003; Uchida et al., 2001; Zeller et al., 2003). Furthermore, SLY1 was shown to be phosphorylated in a PKC-dependent fashion upon B and T cell receptor engagement (Astoul et al., 2003). However, the role of SLY1 for the generation of immune responses was not known so far. To investigate the physiological functions of SLY1 *in vivo,* we generated *Sly1-deficient* mice by gene targeting **(Fig. 1b).** Consistent with the reported localization of the *Slyl* gene on the X chromosome (Beer et al., 2001), splenocytes obtained from male mice hemizygous for the mutant allele did not express detectable levels of *Sly1* mRNA **(Fig. 1c).** Mutant *Sly1-deficient* mice were born at normal Mendelian ratios, were fertile, and did not exhibit any gross anatomical or behavioral abnormalities (data not shown).

In a first set of experiments, the influence of SLY1 expression on lymphoid organogenesis was examined. Analysis of different lymphoid organs revealed that total cell numbers in thymus, spleen, peripheral lymph nodes, and Peyer's patches were significantly reduced by about 33-60% in *Sly1*-deficient mice when compared to wild-type littermates, whereas cellularity in bone marrow and peritoneal cavity appeared to be normal (Fig. 2a). In contrast to peripheral lymph nodes, the lack of SLY1 expression did not affect cell numbers in mesenteric lymph nodes. Interestingly, organogenesis of peripheral and mesenteric lymph nodes requires distinct developmental triggers as was demonstrated by previous work investigating lymphotoxin-β-deficient mice (Koni et al., 1997; Alimzhanov et al., 1997) or mice treated wit lymphotoxin-β receptor fusion protein during gestation (Rennert et al., 1996). In addition to reduced cellularity, the number of macroscopically visible Peyer's patches was significantly lower in *Sly1*-deficient mice than in wild-type littermate controls **(Fig. 2b).** When examined by immunohistochemistry, lymphoid organs of *Sly1*-deficient mice exhibited a normal distribution of B and T lymphocytes as well as non-lymphoid cells such as macrophages and dendritic cells (data not shown).

To further characterize the effects of Slyl-deficiency on the development of T and B cells we performed flow cytometry analyses of various lymphoid organs. The results depicted in **Table 1 and Figure 2c** show that the $CD4^+$ subset of T cells in spleen and lymph nodes was reduced in *Sly1*-deficient as compared to wildtype mice. In contrast, the proportion of CD8 T cells exhibited a relative increase in *Sly1*-null mice. Based on absolute numbers, Slyl-deficient CD4 but not CD8 T cells were significantly reduced in these organs (data not shown). The subset of CD4 T cells expressing CD25, which is known to include regulatory T cells (Maloy and Powrie, 2001; Sakaguchi, 2004; Shevach, 2002), was not altered in *Sly1*-null mice **(Table 1).** In contrast to peripheral lymphoid organs, the ratios of CD4 and CD8 T cell subsets in thymus were not influenced by *Sly1* deficiency. To further investigate T cell development, the T cell receptor Vβ repertoire was determined in thymocytes and lymph node T cells. The results in **Table 2** demonstrate that the Vβ repertoire did not significantly differ between *Sly1*-deficient and wildtype mice.

**Table 1**

T and B cell development in *Sly1*-deficient mice

| Organ | Subset | % positive cells: wildtype | *Sly1–/–* | *p* value |
|---|---|---|---|---|
| Thymus | $CD4^+CD8^+$ | 80.2±3.9 | 75.2±7.9 | 0.13 |
| | $CD4^+CD8^-$ | 10.8±1.5 | 11.5±3.3 | 0.57 |
| | $CD8^+CD4^-$ | 3.4±1.1 | 4.4±1.8 | 0.21 |
| Bone marrow | $B220^{lo}IgM^-$ | 29.9±6.0 | 25.0±6.4 | 0.29 |
| | $B220^{lo}IgM^{lo}$ | 11.3±6.0 | 13.8±7.3 | 0.61 |
| | $B220^{hi}IgM^{hi}$ | 7.0±2.5 | 4.9±2.5 | 0.26 |
| Lymph nodes | $CD4^+CD8^-$ | 49.8±3.2 | 40.8±9.6 | *<0.05* |
| | $CD8^+CD4^-$ | 27.7±4.1 | 36.8±4.0 | *<0.01* |
| Spleen | $CD3^+CD4^+$ | 61.0±1.8 | 49.2±3.0 | *<0.001* |
| | $CD3^+CD8^+$ | 32.9±1.5 | 39.7±2.4 | *<0.001* |
| | $CD4^+CD25^+$ | 2.0±0.2 | 2.0±0.1 | 0.86 |
| | $IgM^{lo}IgD^{hi}$ | 32.3±8.6 | 29.1±11.8 | 0.62 |
| | $IgM^{hi}IgD^{lo}$ | 6.2±2.1 | 2.7±1.0 | *<0.01* |
| | $IgM^+CD21^{hi}CD23^{lo}$ | 9.5±1.9 | 2.9±0.7 | *<0.001* |
| Peritoneal cavity | $IgM^{hi}IgD^{lo}$ | 16.0±2.3 | 17.2±5.0 | 0.79 |

Data are derived from at least 6 independent mice per group.

**Table 2**

Vβ repertoire of Slyl-deficient T cells

| | Thymus | | Lymph nodes | |
|---|---|---|---|---|
| **Vβ repertoire** | **wt** | ***Sly1-l-*** | **wt** | ***Sly1-l-*** |
| $CD3^+V\beta2^+$ | 6.2±0.5 | 5.1±0.4 | 6.4±0.2 | 6.6±0.2 |
| $CD3^+V\beta3^+$ | 3.0±0.4 | 3.3±0.4 | 4.4±0.6 | 3.7±0.2 |
| $CD3^+V\beta4^+$ | 6.4±0.5 | 6.7±0.4 | 6.8±0.5 | 5.8±0.2 |
| $CD3^+V\beta5^+$ | 8.2±0.2 | 7.9±0.9 | 6.9±0.2 | 8.6±0.8 |
| CD3+Vβ6+ | 6.7±0.5 | 5.7±0.6 | 7.9±0.1 | 7.5±0.1 |
| $CD3^+V\beta7^+$ | 2.9±0.4 | 2.9±0.6 | 4.7±0.5 | 5.9±1.0 |
| $CD3^+V\beta8.1/8.2^+$ | 14.0±1.3 | 13.8±0.7 | 16.6±0.1 | 16.6±0.3 |
| $CD3^+V\beta8.3^+$ | 5.6±0.7 | 5.5±0.5 | 5.7±0.1 | 6.6±0.4 |

Table continued
Vβ repertoire of Slyl-deficient T cells

| | Thymus | | Lymph nodes | |
|---|---|---|---|---|
| Vβ repertoire | wt | *Sly1-l-* | wt | *Sly1-l-* |
| $CD3^+V\beta9^+$ | 1.5±0.2 | 1.9±0.6 | 1.8±0.01 | 2.6±0.8 |
| $CD3^+V\beta10b^+$ | 4.3±0.4 | 3.8±0.1 | 4.9±0.2 | 4.9±0.1 |
| $CD3^+V\beta11^+$ | 4.4±0.3 | 4.9±0.8 | 6.0±0.01 | 5.9±0.2 |
| $CD3^+V\beta12^+$ | 3.9±0.2 | 3.9±0.3 | 3.3±0.1 | 3.5±0.1 |
| $CD3^+V\beta13^+$ | 2.1±0.3 | 2.0±0.3 | 2.8±0.1 | 2.8±0.03 |
| $CD3^+V\beta14^+$ | 5.6±0.3 | 6.1±0.3 | 6.4±0.02 | 5.6±0.02 |
| $CD3^+V\beta17a^+$ | <0.1 | <0.1 | n.d. | n.d. |

Data are presented as % positive cells and are derived from 6 independent mice per group. Differences of values for wildtype and *Sly1*-deficient mice were not statistically significant. n.d., not done.

Within the B cell lineage, *Sly1* deficiency caused a marked reduction in the fraction of splenic marginal zone B cells, which were defined by their $IgM^{hi}IgD^{lo}$ or $IgM^+CD21^{hi}CD23^{lo}$ phenotype. Considering the smaller spleen size of Slyl-null mice, the absolute numbers of splenic marginal zone B cells were reduced by about 80% when compared to wildtype controls. In contrast, splenic follicular $IgM^{lo}IgD^{hi}$ B cells as well as peritoneal B1 B cells were present in normal relative numbers in *Sly1*-deficient as compared to wildtype mice (**Table 1).** Moreover, the proportions of precursor B cell subsets in bone marrow were not influenced by *Sly1* deficiency. Thus, genetic ablation of *Slyl* resulted in a marked hypoplasia of various lymphoid organs and preferentially impaired the development and/or maintenance of splenic marginal zone B cells and the peripheral CD4 T cell subset.

**Example 3: Impaired surface Ig-mediated B cell activation and humoral immune responses in *Sly1* -deficient mice**

Genetic ablation of *Slyl* was found to affect the size and composition of the peripheral B cell pool. To determine whether *Sly1* deficiency may also influence B cell function, the B cell antigen receptor was crosslinked by antibodies to IgM and cell proliferation was measured. Under these stimulatory conditions, proliferation is mostly mediated by follicular B cells (Martin and Kearney, 2002). The results in **Figure 3a** show that anti-IgM-induced proliferation of *Sly1*-deficient splenic **B** cells was severely compromised as compared to wildtype **B** cells. Proliferation of **B** cells from *Sly1*- null mice was also impaired when cells received additional stimulation by IL-4. Addition of IL-4 augmented the proliferative response of Slyl-deficient and wildtype B cells to a similar extent suggesting that SLY1 does not affect cell activation through the IL-4 receptor **(Fig. 3a).** Moreover, wild-type and Slyl-deficient B cells exhibited comparable proliferation in response to LPS, which is known to engage Toll-like receptor 4 **(Fig. 3a).** These results suggest that SLY1 is specifically required for *in vitro* activation of B cells through their antigen receptor.
Based on these results, it was next examined whether *Slyl* deficiency would affect basal immunoglobulin levels in serum of mice. The results in **Figure 3b** demonstrate that serum levels of IgM and IgG1 were significantly lower in *Sly1*-deficient mice than in wild-type littermates. In contrast serum levels of IgG2a, IgG3, and IgA isotypes were not significantly altered in the absence of SLY1.

Mice deficient for *Sly1* exhibit a marked reduction of marginal zone B cells **(Table 1, Fig. 2c),** which are known to play an important role in immune responses to T-independent antigens (Martin and Kearney, 2002; Fagarasan and Honjo, 2000). Therefore examined T cell-independent humoral immune responses after immunizing mice with TNP-Ficoll was examined. The results in **Figure 4a** demonstrate that production of TNP-specific IgM antibodies was severely reduced in *Sly1*-deficient mice as compared to wild-type littermates at all time points studied. At the peak of the antibody response (d7), antigen-specific IgM levels were reduced in Slyl-deficient mice by greater than 80%. In addition, TNP-Ficoll induced the production of antigen-specific IgG3 antibodies in wildtype controls. Notably, the IgG3 response was also substantially attenuated in *Sly1*-deficient mice **(Fig. 4a).**

Mice deficient for *Sly1* exhibited reduced basal levels of IgG1, whose production is dependent on T cell help to B cells. It was therefore investigated whether *Sly1* deficiency might also influence T cell-dependent humoral immune responses. Mice were immunized with TNP-CGG adsorbed to alum as an adjuvant and TNP-specific antibodies were measured. The results in **Figure 4b** demonstrate that the levels of IgG1 as well as IgG2a antibodies against TNP were substantially reduced in *Sly1*-deficient mice as compared to wildtype littermate controls. Following TNP-CGG immunization very low levels of antigen-specific IgM antibodies were detected in both groups of mice (data not shown). The results are consistent with the impaired activation of *Sly1*-deficient B cells by antigen receptor ligation and indicate that both T-dependent and -independent humoral immune responses are substantially attenuated in Slyl-deficient mice.

**Example 4: SLY1 is required for antigen receptor-mediated activation of T cells**

Lack of SLY1 expression affected the size and composition of the peripheral T cell pool and impaired T-dependent antibody responses. To directly investigate the influence of *Sly1* deficiency on T cell responses, we stimulated splenic T cells *in vitro* by incubation with antibodies to CD3. The results in **Figure 5a** demonstrate that the production of IL-2, IL-10, TNF, and the Th1- and Th2-type cytokines IFN-γ and IL-4 by *Sly1*-deficient splenocytes was severely impaired when compared to wild-type cells. Cytokine production was reduced to a similar extent when Slyl-deficient cells were activated in the presence or absence of anti-CD28 antibodies suggesting that costimulation through CD28 cannot compensate for *Sly1* deficiency. In contrast to T cell receptor ligation, stimulation with PMA and calcium ionophore resulted in a comparable production of IL-2 by wildtype and Slyl-deficient T cells **(Fig. 5b).**

In additional experiments, the proliferative response of splenic T cells stimulated with anti-CD3 antibodies was examined. The dose-response curve depicted in **Figure 6a** demonstrates that, at all concentrations tested, T cell proliferation was significantly attenuated by genetic ablation of *Sly1.* CD3-driven proliferation of *Sly1*-deficient T cells was also markedly impaired in the presence of anti-CD28 antibodies. In contrast to CD28 costimulation, the addition of exogenous IL-2 compensated for the lack of SLY1 expression resulting in comparable proliferation of *Sly1*-deficient and wildtype T cells **(Fig. 6a).** Because IL-2 activity requires CD25 expression, cell surface levels of CD25 were measured following CD3 stimulation. The results in **Figure 6b** demonstrate that upregulation of CD25 was comparable between wildtype and *Sly1*-deficient CD4 and CD8 T cells.

Similarly, *Sly1* deficiency did not influence CD3-stimulated expression of CD69 **(Fig. 6c).** Together, these results suggest that *in vitro* T cell proliferation in response to antigen-receptor ligation is markedly impaired in the absence of SLY1 and that this defect may be attributed, at least in part, to reduced IL-2 production.

The role of SLY1 for T cell responses was further investigated in allogeneic mixed lymphocyte reactions using irradiated BALB/c splenocytes as stimulator cells. As shown in **Figure 7a** the proliferation of *Sly1*-deficient T cells was significantly reduced as compared to wildtype T cells. To further distinguish the effects of *Sly1* deficiency on CD4 and CD8 T cell subset, purified CD4 and CD8 T cells were used as responder cells. Lack of SLY1 expression severely attenuated the proliferative response of CD4 T cells **(Fig. 7b),** whereas CD8 T cell proliferation was only moderately affected **(Fig. 7c).** To investigate the function of SLY1 for CD8 T cells in more detail, mice were immunized with ovalbumin and CpG-DNA as adjuvant and cytotoxic T cell activity was determined *in vitro.* Under these conditions of immunization, the generation of antigen-specific cytotoxic T cells is independent of CD4 T cells (Sparwasser et al., 2000; Cho et al., 2000). The results in **Figure 7d** show that Slyl-deficient mice and wildtype controls were able to mount comparable cytotoxic T cell activity. These results therefore indicate that SLY1 is essential for the proliferation and induction of certain effector functions of T cells with more pronounced effects on the CD4 cell subset.

**Example 5: Prolonged allograft survival in *Sly1*-deficient mice**

Sly1 deficiency caused impaired T cell cytokine production and attenuated the proliferative response of T cells to allogeneic stimulator cells *in vitro.* To investigate whether the lack of SLY1 may also affect T cell responses *in vivo,* the role of SLY in a model of heterotopic heart transplantion was examined. The results in **Figure 8a** demonstrate that *Sly1*-deficient mice (H-$2^b$) rejected fully allogeneic heart transplants (H-$2^d$) as efficiently as wildtype hosts (H-$2^b$). Control syngeneic grafts were not rejected during the observation period.

The activation of alloreactive T cells, which mediate acute transplant rejection, involves multiple pathways that act, at least in part, in a redundant fashion. During rejection of fully allogeneic grafts, help provided by activated NK and/or NKT cells is sufficient to overcome defects of T cell activation such as those caused by CD28 deficiency (Maier et al., 2001). Thus, the influence of an individual pathway of T cell activation on transplant rejection may only become apparent in the absence of help derived from NK-receptor-bearing cells. Because recognition of self MHC class I molecules is known to prevent activation of NK cells, we transplanted cardiac allografts semi-identical in haplotype (H-$2^{bXd}$) into wildtype and *Sly1-deficient* mice (both H-$2^b$). Consistent with our previous work (Maier et al., 2001), semi-identical H-$2^{bxd}$ transplants were acutely rejected by H-$2^b$ wildtype hosts **(Fig. 8b).** In marked contrast, transplant survival was significantly prolonged by genetic ablation of *Slyl* **(Fig. 8b).** Half of the semi-identical hearts transplanted into Slyl-deficient hosts survived longer than 60 days and half of the grafts were rejected with delayed kinetics as compared to wildtype hosts. These results therefore document that *Slyl* deficiency substantially improves acceptance of cardiac allografts in the absence of NK cell help.

Recent experimental evidence obtained from mice and cell lines rendered genetically deficient for the expression of leukocyte-specific adaptor proteins indicates that their function is crucial for lymphocyte development and antigen receptor responses (Kurosaki, 2002; Jordan et al., 2003; Samelson, 2002).

The present report documents an essential function for SLY1 in transplantation rejection. SLY1 is a member of a novel family of SH3 and SAM domain proteins. SLY1 was found to be crucial for antigen receptor-induced proliferation and cytokine production of T cells, the generation of marginal zone B cells, the proliferation of B cells and generation of humoral immune responses, and the rejection of cardiac allografts. In contrast, SLY1 appears to be dispensable for IL-2 receptor-mediated responses of T cells and B cell activation through TLR4.

In *Sly1*-deficient mice, the antibody response to T-dependent as well as T-independent antigens was severely impaired thereby identifying SLY1 as a novel molecular regulator of humoral immune responses. T-dependent antibody responses may be affected by *Sly1* deficiency at multiple levels. Efficient B cell activation by T-dependent antigens requires signals delivered through binding of the antigen to the B cell receptor as well as generation antigen-specific T helper cells that provide costimulatory signals to B cells through CD40-CD40L interactions and the secretion of cytokines. In this invention, it is surprisingly demonstrated that proliferative responses of B and T cells following antigen receptor ligation were markedly diminished by *Sly1* deficiency. Generation of sufficient numbers of antigen-specific antibody forming cells as well as T helper cells through antigen receptor-driven clonal expansion is impaired in the absence of SLY1. In addition, *Sly1-* deficient T cells exhibited reduced secretion of cytokines that are required for costimulation of proliferation and isotype switching by antigen-reactive B cells. Consistent with a diminished production of IL-4 and IFN-γ, both IgGI and IgG2a responses to T-dependent antigen were diminished in Slyl-null mice.

Generation of T-dependent antibody responses was thought to be mediated mainly by follicular B2 B cells. However, recent work demonstrated that marginal zone B cells are functionally heterogeneous and rapidly produce large numbers of antibody-forming cells (Song and Cerny, 2003). Moreover, these cells give rise to germinal centers with characteristic somatic hypermutation and generate immunological memory. In *Sly1*-deficient mice, diminished absolute numbers of splenocytes and a preferential loss of marginal zone B cells lead to a reduction of this B cell subset by about 80%. The defect in marginal zone B cell generation may therefore provide an additional explanation for the impaired antibody production against T-dependent antigen in the absence of SLY1.

T-independent antibody responses are considered important for host defenses as they bridge early innate with relatively late appearing adaptive immune reactions (Fagarasan and Honjo, 2000). In addition to B1 B cells, marginal zone B cells play a major role for the first-line responses against blood-borne T-independent antigens. Evidence for this notion was provided by analysis of *Pyk-2*-deficient mice, which were found to have a selective defect in marginal zone B cell development that was associated with severely impaired T-independent antibody responses (Guinamard et al., 2000). Subsequent studies with B cell receptor transgenic mice directly demonstrated that marginal zone B cells efficiently generate antigen-specific plasmablasts after immunization with T-independent antigen (Martin et al., 2001). Because *Sly1* deficiency severely impaired generation of marginal zone B cells, it appears likely that the impaired T-independent antibody response in *Sly1*-deficient mice may result, at least in part, from reduced numbers of antigen-responsive marginal zone B cells. The finding that total splenic B cells show reduced proliferation in response to ligation of surface IgM suggests that attenuated B cell receptor-mediated activation of antigen-specific marginal zone B cells and possibly B1 B cells may also play a role.

Newly formed surface $IgM^+$ B cells that are generated in the bone marrow migrate to peripheral lymphoid organs such as spleen to complete maturation (Rolink et al., 2004). Experiments involving the inducible ablation of the B cell receptor indicate that all B cells may constantly receive signals of unknown molecular identity from their environment leading to engagement of the B cell receptor and B cell survival (Lam et al., 1997). The enrichment of particular B cell idiotypes in the marginal zone and alterations of marginal zone B cell development in mice lacking positive or negative regulators of B cell receptor signaling suggested that relatively strong signals initiated by the B cell receptor drive the generation of marginal zone B cells (Allman et al., 2004; Martin and Kearney, 2002). Thus, genetic ablation of CD19, *Btk,* or the p1108 subunit of PI3-kinase, all of which promote B cell receptor signaling, impairs generation and/or positive selection of marginal zone B cells (Otero et al., 2003; Clayton et al., 2002; Martin and Kearney, 2000), while PTEN deficiency, which results in enhanced B cell receptor signaling, is characterized by increased numbers of marginal zone B cells (Anzelon et al., 2003). The present report documents a distinct role of SLY1 for B cell responses and demonstrates a substantial reduction of antigen receptor-driven proliferation of *Sly1*-deficient B cells that is not reversed by costimulation with IL-4. It is therefore conceivable that attenuated B cell receptor signals lead to the preferential reduction of marginal zone versus follicular B cell numbers in *Sly1*-deficient mice. However, a mere reduction of B cell receptor signal intensity in the absence of SLY1 does to explain these observations, because genetic inactivation of regulators of B cell receptor signaling also affects development of B1 B cells (Anzelon et al., 2003; Otero et al., 2003; Clayton et al., 2002; Engel et al., 1995; Rickert et al., 1995), whereas *Sly1*-deficient mice have normal B 1 cell numbers.

The present invention demonstrates that SLY1 is not only important for the generation of humoral immune responses, but also reveals a critical role of SLY1 for allograft rejection in a model of heterotopic heart transplantation. CD4 T cells are known to be of paramount importance for allograft rejection (Rosenberg and Singer, 1992). Activated CD4 T cells direct the immune response against allografts by secreting cytokines that activate and attract effector cells such as CD8 T cells, B cells, and NKT cells (Walsh et al., 2004). Without being bound by theory, the surprising prolonged survival of semi-identical allografts in *Sly1*-deficient mice may be explained, at least in part, by the fact that *Sly1*-deficiency leads to an attenuated proliferation and cytokine production of T cells upon antigen receptor ligation and an impaired capacity of CD4 T cells to respond to an allogeneic stimulus.

Activation of allograft-reactive CD4 T cells may occur through multiple pathways that involve an intense interplay of innate and adaptive immune reactions. Thus, NK-receptor-bearing cells become activated upon transplantation of fully allogeneic grafts and provide help to T cells, which subsequently mediate acute transplant rejection (Maier et al., 2001). Importantly, help provided by activated NK and/or NKT cells may be sufficient to overcome defects of T cell costimulation such as those caused by CD28 deficiency (Maier et al., 2001). The pathways for the induction of T cell responses to allografts are, at least in part, functionally redundant. Therefore, the influence of individual regulators of T cell activation (i.e. SLY1) on transplant rejection may only become apparent after reducing this complexity. To avoid help derived from NK-receptor-bearing cells, semi-identical (H-$2^{bxd}$) cardiac allografts into wildtype and Slyl-deficient mice (both H-$2^{b}$) were transplanted. It is shown that the acceptance of semi-identical grafts was substantially extended in *Sly1*-deficient hosts with 50% of the grafts surviving even after 60 days. Fully allogeneic grafts, however, were vigorously rejected in *Sly1*-deficient mice. The phenotype of *Sly1*-deficient mice in transplantation is therefore similar to that of CD28-null mice, which also accept semi-identical cardiac transplants but, in contrast to the situation described here, efficiently reject fully allogeneic grafts.

The X chromosome harbors numerous genes that are inactivated in genetic immune disorders. The *Sly1* gene is located in close proximity to genes encoding CD40L and SH2D1A. SH2D1A mutations result in X-linked lymphoproliferative disease, while CD40L deficiency causes X-linked hyper-IgM syndrome (Coffey et al., 1998; Renshaw et al., 1994; Xu et al., 1998). Other examples of X chromosomal immune response genes include *Btk* mutations, which result in X-linked agamma-globulinemia (Tsukada et al., 1993; Vetrie et al., 1993). In addition, there are also several X-linked immune diseases for which the corresponding genetic defects have not yet been identified. The results of the present study demonstrate that genetic ablation of *Slyl* causes multiple defects in B and T cell responses. *Sly1* deficiency may therefore represent a novel type of X-linked immunodeficiency specifically affecting adaptive immunity.

## Additional literature

Alimzhanov, (1997). Proc. Natl. Acad. Sci. U. S. A. 94, 9302-9307.
Allman, (2004). Immunol. Rev. *197,* 147-160.
Anzelon, (2003). Nat. Immunol. 4, 287-294.
Astoul, (2003). J. Biol. Chem. 278, 9267-9275.
Beer, (2001). Biochim. Biophys. Acta *1520,* 89-93.
Cariappa, (2001) Immunity 14, 603-615.
Cho, (2000) Nat. Biotech. 18, 509-514.
Claudio, (2001) Oncogene 20, 5373-5377.
Clayton, (2002) J. Exp. Med. 196, 753-763.
Coffey, (1998) Nat. Genet. 20 , 129-135.
Engel, (1995) Immunity 3, 39-50.
Fagarasan, (2000) Science 290, 89-92.
Freedman, (2003) Nat. Struct. Biol. *10,* 504-512.
Garside, (1998) Science *281,* 96-99.
Guinamard, (2000) Nat. Immunol. 1, 31-36.
Jordan, (2003) Nat. Immunol. 4, 110-116.
Koni, (1997) Immunity 6, 491-500.
Kurosaki, (2002) Nat. Rev. Immunol. 2, 354-363.
Lam, (1997) Cell 90, 1073-1083.
Maier, (2001) Nat. Med. 7, 557-562.
Maloy, (2001) Nat. Immunol. 2, 816-822.
Martin, (2000) Immunity 12, 39-49.
Martin, (2002) Nat. Rev. Immunol. 2, 323-335.
Martin, (2001) Immunity 14, 617-629.
Murphy, (2002) Nat. Rev. Immunol. 2, 933-944.
Otero, (2003) J. Immunol. *170,* 73-83.
Re, (2003) Cancer Res. 63, 2606-2609.
Rennert, (1996) J. Exp. Med. 184, 1999-2006.
Renshaw, (1994) J. Exp. Med. 180, 1889-1900.
Rickert, (1995) Nature 376, 352-355.

Rolink, (2004) Immunol. Rev. 197, 41-50.
Rosenberg, (1992) Annu. Rev. Immunol. 10, 333-358.
Sakaguchi, (2004) Annu. Rev. Immunol. 22, 531-562.
Samelson, (2002) Annu. Rev. Immunol. 20, 371-394.
Shevach, (2002) Nat. Rev. Immunol. 2, 389-400.
Simeoni, (2004) Curr. Opin. Immunol. 16, 304-313.
Song, (2003) J. Exp. Med. 198, 1923-1935.
Sparwasser, (2000) Eur. J. Immunol. 30, 3591-3597.
Tsukada, (1993) Cell 72, 279-290.
Uchida, (2001) Biochem. Biophys. Res. Commun. 288, 137-141.
Vetrie, (1993) Nature 361, 226-233.
Walsh, (2004) Immunity 20, 121-131.
Xu, (1998) Immunity *1*, 423-431.
Zeller, (2003) Oncogene 22, 2972-2983.

## Claims

1. A pharmaceutical composition comprising an inhibitor/negative regulator/antagonist of SLY-1 (SH3 domain protein expressed in lymphocytes).

2. Use of an inhibitor/negative regulator/antagonist of SLY-1 for the preparation of a pharmaceutical composition for the treatment or prevention of undesired side effects in transplantation of cells, tissues or organs and/or for the treatment of graft-versus-host disease.

3. A method for treating or preventing undesired side effects in transplantation of cells, tissues or organs in a subject comprising administering an inhibitor/negative regulator/antagonist of SLY-1 to mammal in need of such a therapy.

4. The pharmaceutical compositition of claim 1, the use of claim 2 or the method of claim 3, wherein said inhibitor/negative regulator/antagonist is selected from the group consisting of small-binding molecules, proteinaceous binding molecules, intracellular-binding receptors, aptamers, intramers, RNAi (double-stranded RNA), siRNA and anti-SLY-1-antisense molecules.

5. The pharmaceutical composition, use or method of claim 4, wherein said proteinaceous binding molecule is an antibody, an antibody fragment or an antibody derivative.

6. The pharmaceutical composition, use or method of claim 4, wherein said anti-SLY-1-antisense molecule comprises a nucleic acid molecule which is the complementary strand of a reversed complementary strand of the coding region of SLY-1.

7. The pharmaceutical composition, use or method of claim 4, wherein said anti-SLY-1 siRNA is selected from the group consisting of an antisense molecule as shown in any one of SEQ ID NOS:7 to 16.

8. The pharmaceutical composition, use or method of any of claims 1 to 7, wherein said SLY-1 is selected from the group consisting of a SLY-1-molecule selected from the group consisting of:

(a) a SLY-1 molecule encoded by a nucleic acid molecule comprising a nucleic acid molecule as shown in any one of SEQ ID NO: 1, SEQ ID NO: 3 or SEQ ID NO: 5;
(b) a SLY-1 molecule encoded by a DNA molecule coding for a polypeptide having an amino acid sequence as shown in SEQ ID NO: 2; SEQ ID NO: 4 or SEQ ID NO: 6;
(c) a SLY-1 molecule which is encoded by a DNA sequence that hybridizes to the complementary strand of the nucleic acid molecule as defined in (a) or the DNA molecule as defined in (b) and which encodes a polypeptide that is specifically expressed in lymphocytes;
(d) a SLY-1 molecule which comprises an amino acid sequence as shown in SEQ ID NO: 2, 4 or 6;
(e) a SLY-1 molecule which comprises an amino acid sequence which is at least 90% identical to the full-length amino acid sequence as shown in SEQ ID NO: 2, 4 or 6; and
(f) a SLY-1 molecule which is encoded by a DNA sequence which is degenerate to a DNA sequence/nucleic acid molecule as defined in (a) to (c).

**9.** The use or the method of anyone of claims 2 to 8, wherein said transplantation is an autologous, an allogenic, a homeogenic, a syngenic or a xenogenic transplantation.

**10.** A method for screening inhibitors or negative regulators of SLY-1 activity or expression comprising the steps of

(a) contacting a cell or a non-human organism expressing SLY-1 or capable of expressing SLY-1 with a compound to be tested;
(b) determining antigen-receptor signal transductions in the presence of said compound to be tested when compared to a cell not contacted with said compound; and
(c) identifying the compound which inhibits SLY-1 activity.

**11.** The method of claim 10, wherein said determination of antigen-receptor signal transduction comprises the stimulation by antibodies directed against CD3 (anti-CD3 antibodies) or by antibodies directed against IgM (anti-IgM antibodies).

**12.** The method of claim 10 or 11, wherein said determination comprises the determination of the phosphorylation status of SLY-1 in said cell or said organisms.

**13.** The method of claim 12, wherein said determination of the phosphorylation status of SLY-1 comprises the determination of the phosphorylation of a Serine-residue being homologous or identical to Ser27 of SEQ ID NO: 2 or SEQ ID NO: 4.

**14.** A method for screening inhibitors or negative regulators of SLY-1 activity or expression comprising the steps of

(a) contacting a cell or a non-human animal/organism expressing SLY-1 or being capable of expressing SLY-1 with a compound to be tested;
(b) contacting a cell or a non-human animal/organism that does not express SLY-1 or wherein SLY-1 is inactive with said compound to be tested;
(c) evaluating the response of said cells or non-human animals/organisms of (a) and (b) and comparing said responses;
(d) identifying the compound that inhibits SLY-1 activity or expression.

**15.** The method of claim 14, whereby said evaluation step (c) comprises a proliferation assay.

**16.** The method of claim 15, whereby a SLY-inhibitor or negative regulator inhibits or slows down proliferation in SLY-1 positive cells and whereby said inhibitor or negative regulator does not influence proliferation or enhances proliferation in SLY-1 negative cells.

**17.** The method of claim 14, whereby said evaluation step (c) comprises the measurement of cytokine production in lymphocytes.

**18.** The method of claim 17, whereby said lymphocyte is a T-cell and whereby said cytokine is interleukin-2 or interferon-$\gamma$.

**19.** The method of claim 17 or 18, whereby a SLY-1 inhibitor or negative regulator reduces the production of said cytokines.

**20.** The method of any one of claims 10 to 19, wherein said non-human organism is selected from the group consisting of C. elegans, yeast, zebrafish, drosophila, mouse, rat, guinea pig, dog and cat.

**21.** A method for the preparation of a pharmaceutical composition comprising the steps of

(a) identifying a compound capable of inhibiting or negatively-regulating SLY-1 activity by the method of anyone of claims 10 to 20; and
(b) formulating said compound with a pharmaceutically acceptable carrier.

Figure 1

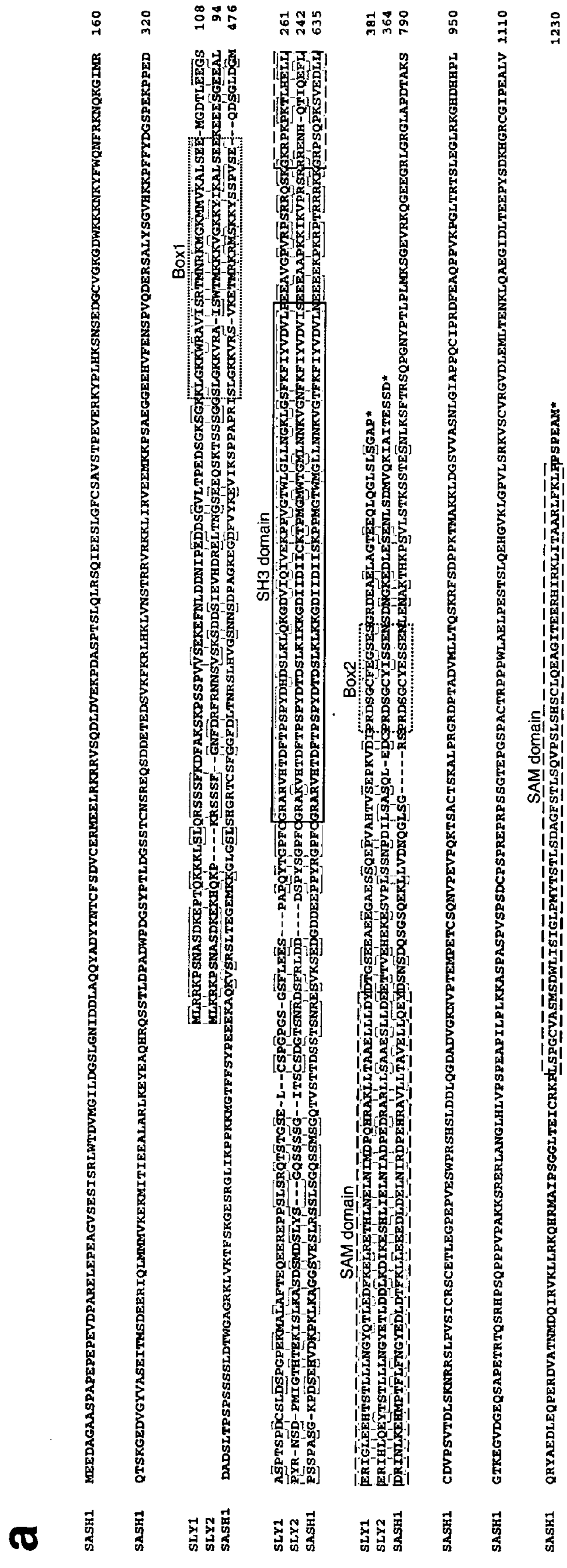

Figure 1

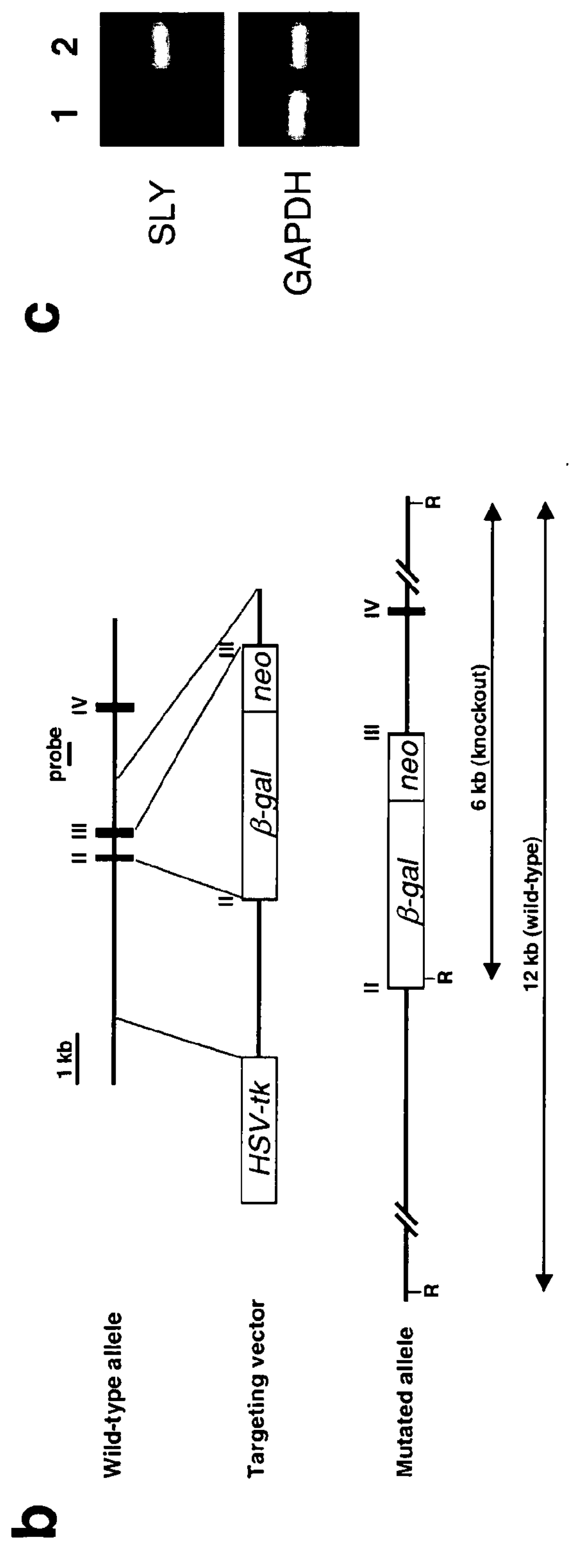
b
Wild-type allele
Targeting vector
Mutated allele
1 kb
probe
II III IV
HSV-tk
β-gal
neo
R
6 kb (knockout)
12 kb (wild-type)
c
1 2
SLY
GAPDH

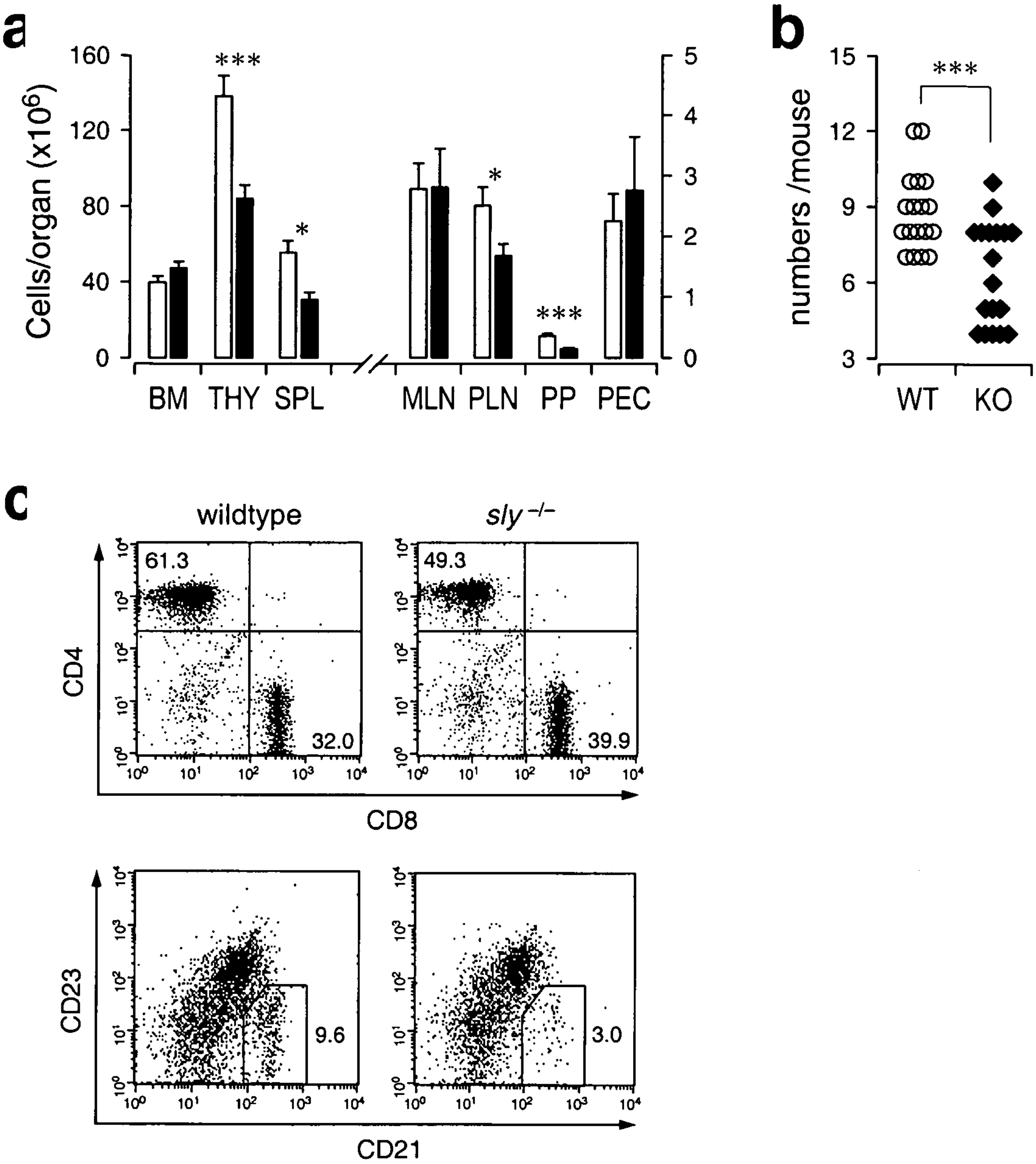
a
Cells/organ (x10⁶)
BM
THY
SPL
MLN
PLN
PP
PEC
b
numbers /mouse
WT
KO
c
wildtype
sly -/-
CD4
CD8
61.3
32.0
49.3
39.9
CD23
CD21
9.6
3.0

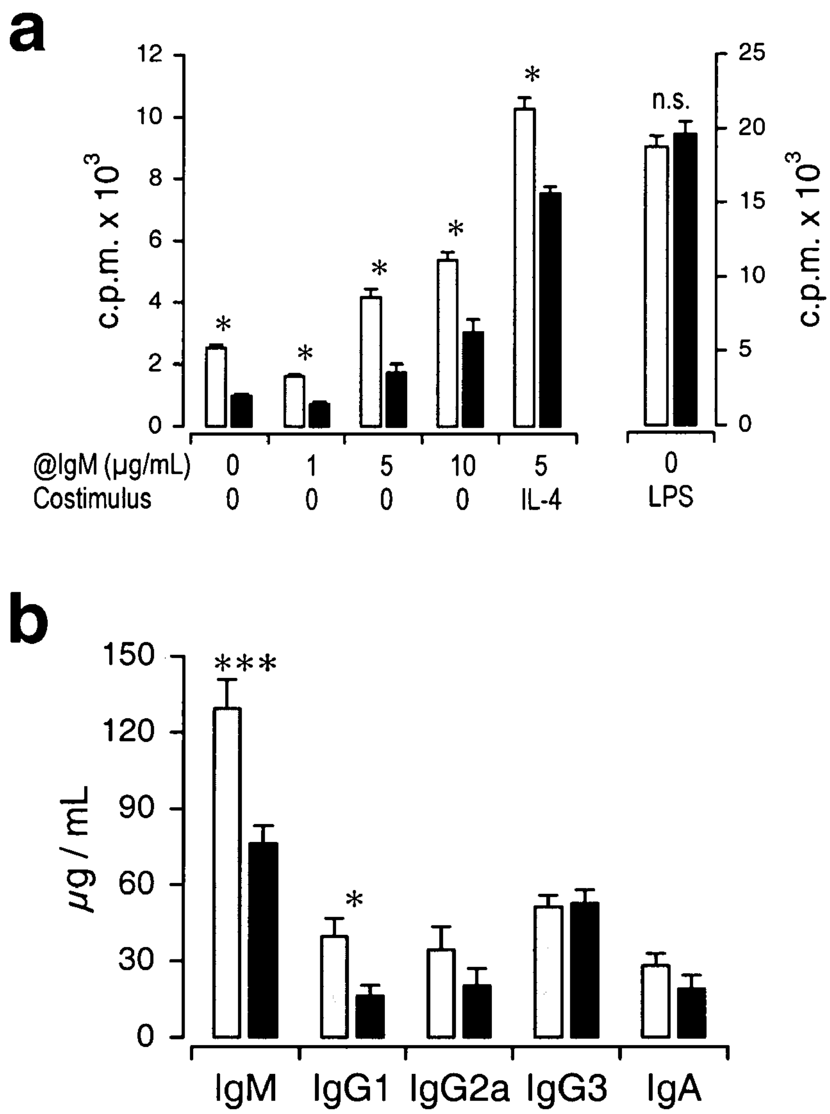
a
c.p.m. x 10^3
12
10
8
6
4
2
0
25
20
15
10
5
0
*
n.s.
@IgM (µg/mL) 0 1 5 10 5 0
Costimulus 0 0 0 0 IL-4 LPS
b
µg / mL
150
120
90
60
30
0
***
IgM IgG1 IgG2a IgG3 IgA

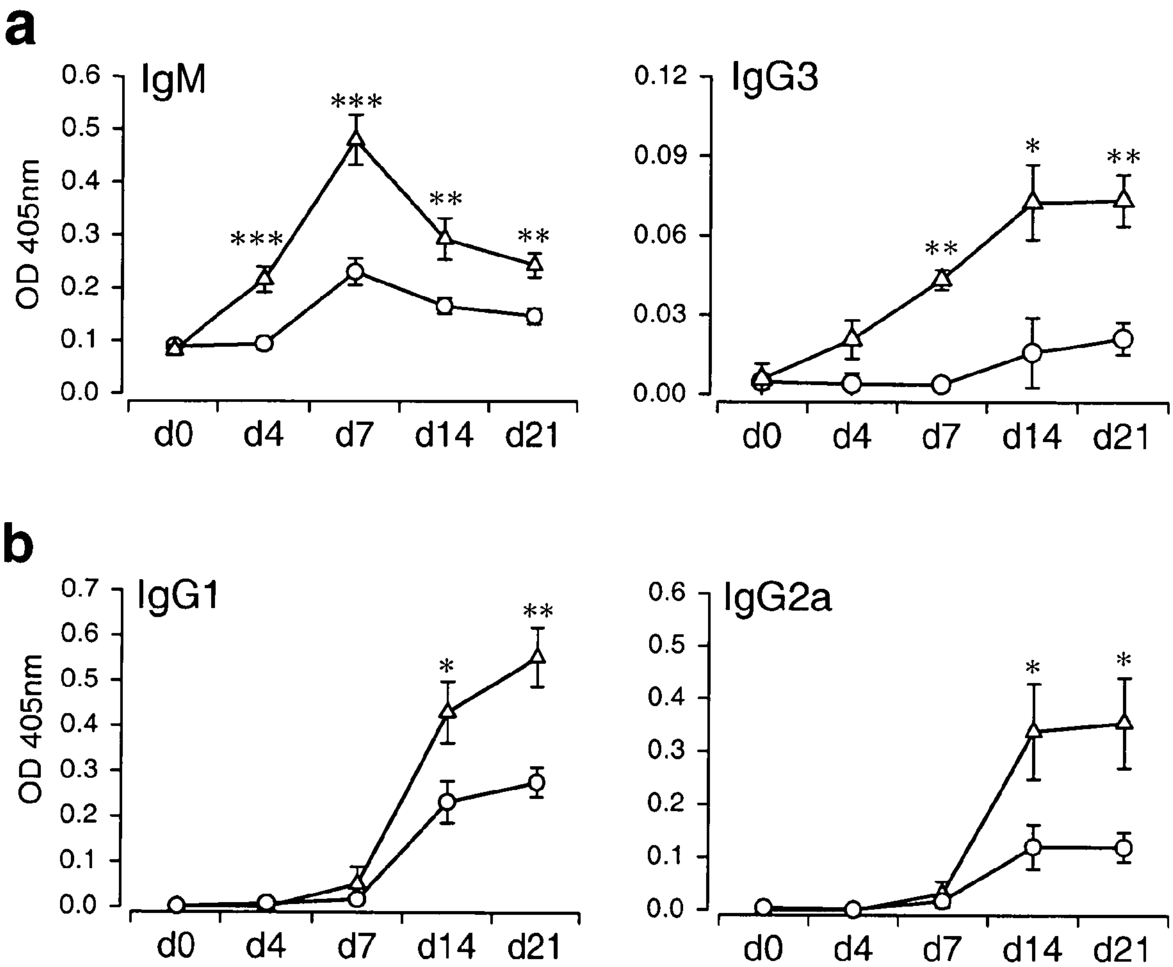
a
IgM
OD 405nm
0.6
0.5
0.4
0.3
0.2
0.1
0.0
***
***
**
**
d0 d4 d7 d14 d21
IgG3
0.12
0.09
0.06
0.03
0.00
*
**
**
d0 d4 d7 d14 d21
b
IgG1
OD 405nm
0.7
0.6
0.5
0.4
0.3
0.2
0.1
0.0
*
**
d0 d4 d7 d14 d21
IgG2a
0.6
0.5
0.4
0.3
0.2
0.1
0.0
*
*
d0 d4 d7 d14 d21

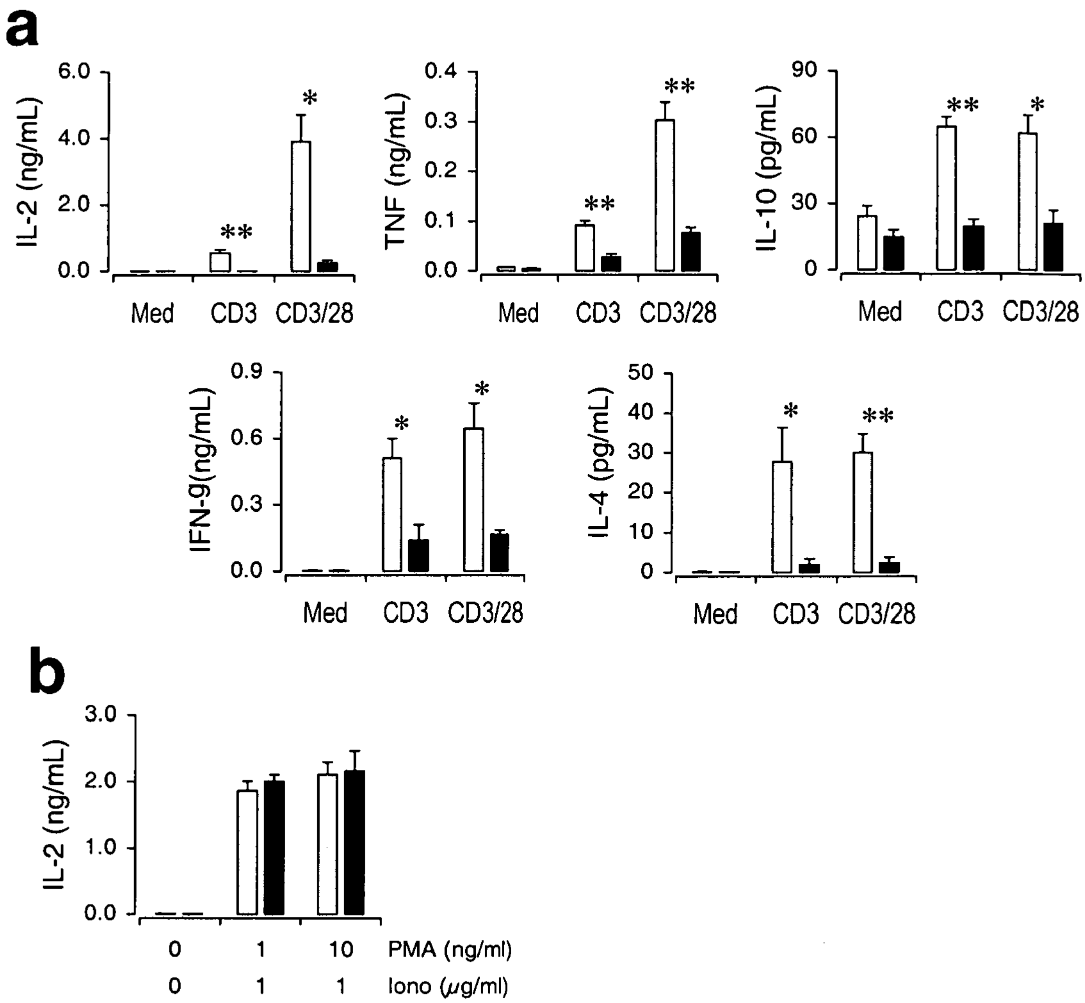
a
IL-2 (ng/mL)
6.0
4.0
2.0
0.0
**
*
Med
CD3
CD3/28
TNF (ng/mL)
0.4
0.3
0.2
0.1
0.0
**
**
Med
CD3
CD3/28
IL-10 (pg/mL)
90
60
30
0
**
*
Med
CD3
CD3/28
IFN-g(ng/mL)
0.9
0.6
0.3
0.0
*
*
Med
CD3
CD3/28
IL-4 (pg/mL)
50
40
30
20
10
0
*
**
Med
CD3
CD3/28
b
IL-2 (ng/mL)
3.0
2.0
1.0
0.0
0
1
10
PMA (ng/ml)
0
1
1
Iono (μg/ml)

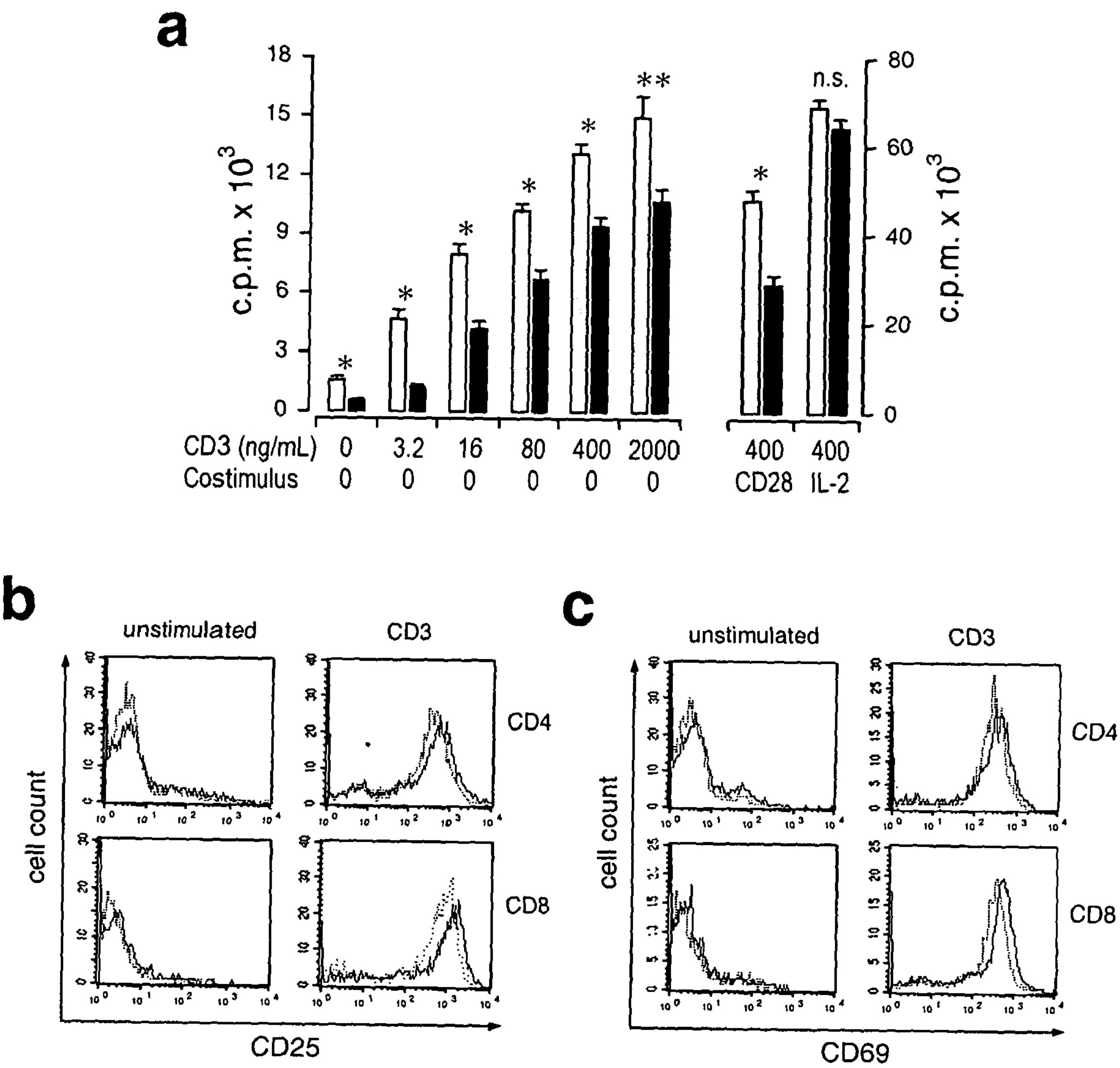
a
c.p.m. x $10^3$
n.s.
CD3 (ng/mL) 0 3.2 16 80 400 2000 400 400
Costimulus 0 0 0 0 0 0 CD28 IL-2
b
unstimulated
CD3
CD4
CD8
cell count
CD25
c
unstimulated
CD3
CD4
CD8
cell count
CD69

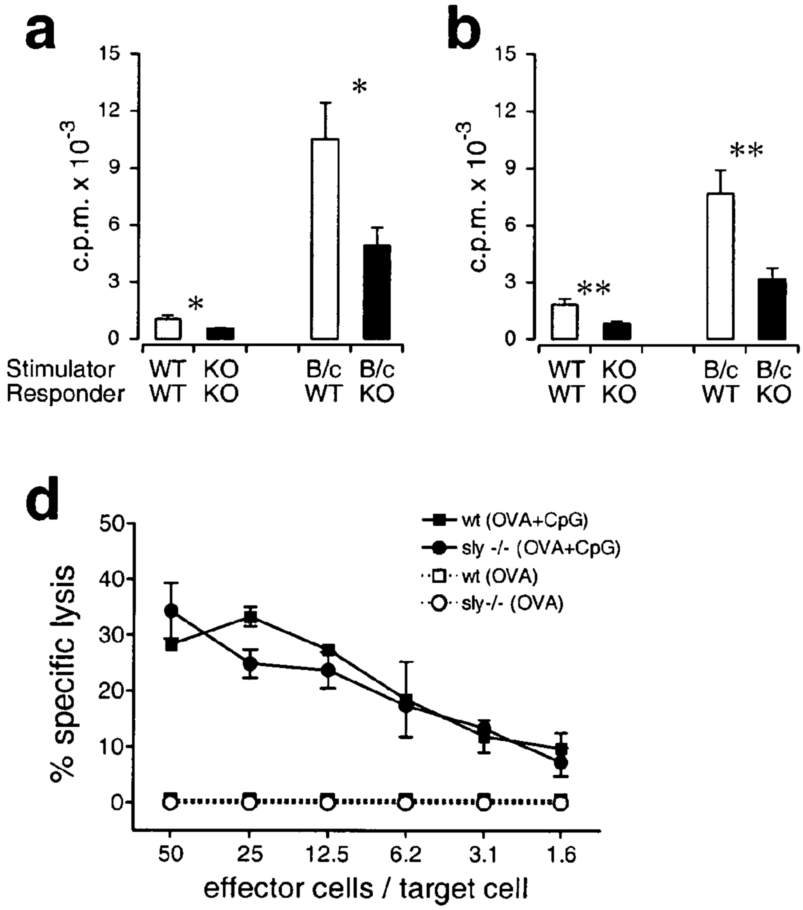
a
b
c
c.p.m. x 10-3
15
12
9
6
3
0
*
**
Stimulator WT KO B/c B/c
Responder WT KO WT KO
d
% specific lysis
50
40
30
20
10
0
wt (OVA+CpG)
sly -/- (OVA+CpG)
wt (OVA)
sly-/- (OVA)
50 25 12.5 6.2 3.1 1.6
effector cells / target cell

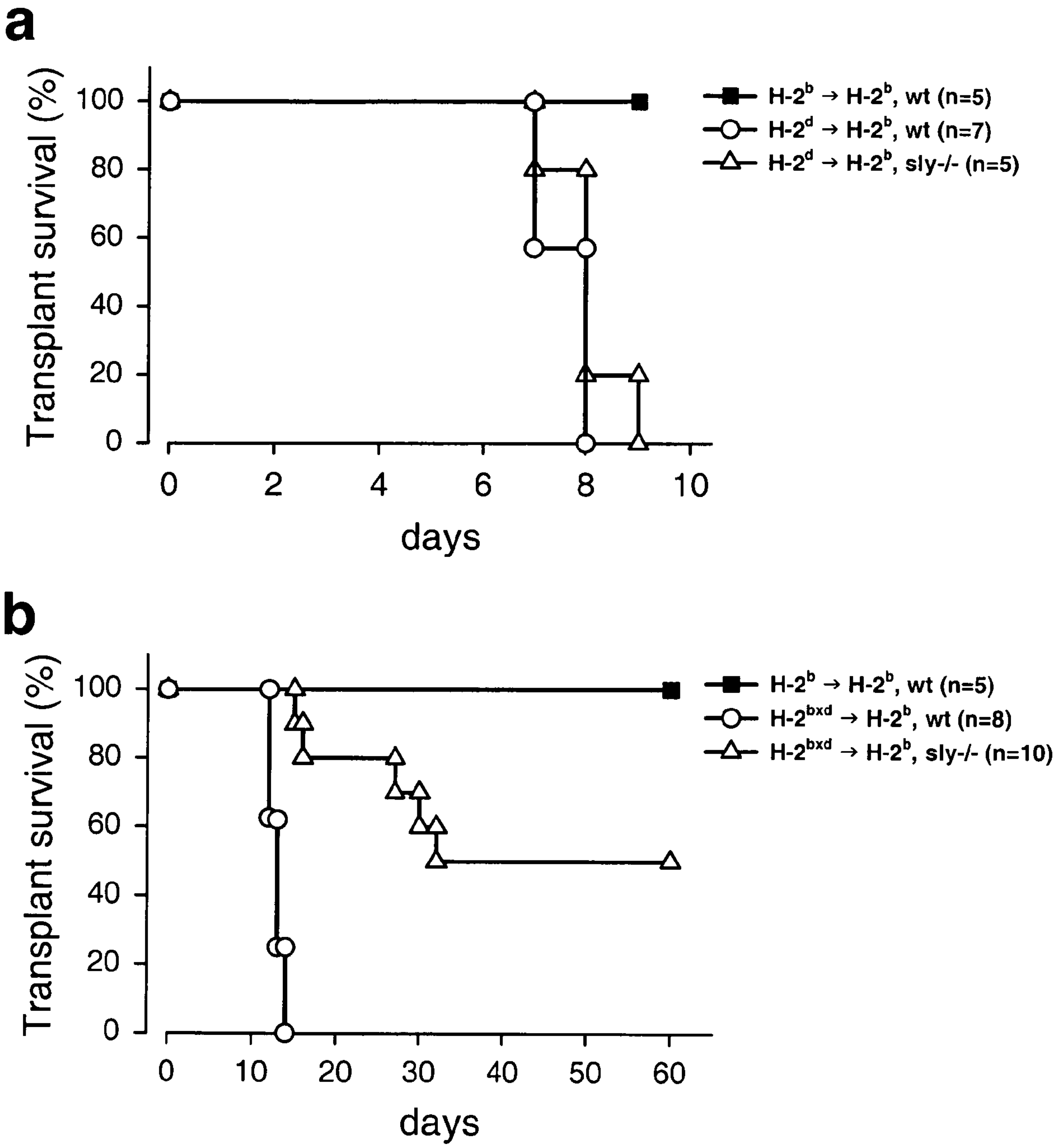
a
Transplant survival (%)
100
80
60
40
20
0
0
2
4
6
8
10
days
H-2b → H-2b, wt (n=5)
H-2d → H-2b, wt (n=7)
H-2d → H-2b, sly-/- (n=5)
b
Transplant survival (%)
100
80
60
40
20
0
0
10
20
30
40
50
60
days
H-2b → H-2b, wt (n=5)
H-2bxd → H-2b, wt (n=8)
H-2bxd → H-2b, sly-/- (n=10)

European Patent Office

## PARTIAL EUROPEAN SEARCH REPORT

which under Rule 45 of the European Patent Convention shall be considered, for the purposes of subsequent proceedings, as the European search report

Application Number EP 04 02 8251

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br>Y | WO 03/083047 A (EXELIXIS INC (US); BELVIN FRANCIS-LANG FRIEDMAN PLOWMAN HEUER LI FUNKE) 9 October 2003 (2003-10-09)<br>SEQ ID NO:44, 100<br>* the whole document *<br>----- | 1,4-6,8, 10,14, 20,21<br>12,13 | C12N15/12<br>C07K14/47<br>C12N15/11<br>A61K31/7088<br>C07K16/18<br>A61K38/00<br>G01N33/50<br>G01N33/68<br>C12Q1/68<br>A61P37/06 |
| Y,D | ASTOUL E. ET AL.: "Approaches to define antigen receptor-induced serine kinase signal transduction pathways"<br>THE JOURNAL OF BIOLOGICAL CHEMISTRY, vol. 278, no. 11, 14 March 2003 (2003-03-14), pages 9267-9275, XP002335439<br>* the whole document *<br>----- | 12,13 | |
| A | NECHIPORUK A. ET AL.: "Positional cloning of a temperature-sensitive mutant emmental reveals a role for Sly1 during cell proliferation in zebrafish fin regeneration"<br>DEVELOPMENTAL BIOLOGY, vol. 258, 2003, pages 291-306, XP002335440<br>* the whole document *<br>-----<br>-/-- | | TECHNICAL FIELDS SEARCHED (Int.Cl.7)<br>C12N<br>C07K<br>A61K<br>G01N<br>C12Q<br>A61P |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 12 July 2005 | Macchia, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C07)

European Patent Office

# PARTIAL EUROPEAN SEARCH REPORT

Application Number
EP 04 02 8251

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A,D | BEER S. ET AL.: "Molecular cloning and characterization of a novel SH3 protein (SLY) preferentially expressed in lymphoid cells"<br>BIOCHIMICA ET BIOPHYSICA ACTA . GENE STRUCTURE AND EXPRESSION, ELSEVIER, AMSTERDAM, NL,<br>vol. 1520, no. 1,<br>30 July 2001 (2001-07-30), pages 89-93, XP004255727<br>ISSN: 0167-4781<br>* the whole document *<br>----- | | |
| | | | TECHNICAL FIELDS SEARCHED (Int.Cl.7) |

2

EPO FORM 1503 03.82 (P04C10)

European Patent Office

**INCOMPLETE SEARCH SHEET C**

Application Number
EP 04 02 8251

Although claims 3-9 are directed to a method of treatment of the human/animal body (Article 52(4) EPC), the search has been carried out and based on the alleged effects of the compound/composition.

-----

Claim(s) searched completely:
5-7,10-20

Claim(s) searched incompletely:
1-4, 8 9, 21

Reason for the limitation of the search:

Present claim 1 relates to a composition defined by reference to a desirable characteristic or property, namely that it acts as an inhibitor/negative regulator/antagonist of SLY-1.
Present claims 2 and 3 relate to a therapeutical use/method of said inhibitor/negative regulator/antagonist of SLY-1.
Present claims 4, 8 and 9 are dependent on at least one of said claims 1-3.

The claims cover all inhibitors/negative regulators/antagonists of SLY-1 having this characteristic or property, whereas the application provides support within the meaning of Article 84 EPC and/or disclosure within the meaning of Article 83 EPC for only a very limited number of such compounds, i.e. compounds obtainable by means of antisense technology and antibodies.

Present claim 21 relates to a method comprising the step of formulating a compound capable of inhibiting or negatively-regulating SLY-1 activity, identified by the method of anyone of claims 10 to 20, with a pharmaceutically aceüptable carrier.

The claim does not give a true technical characterization of said compound. In fact, said claim attempts to define the compound to which it refers in terms of the result to be achieved (EPC Guidelines C-III, 4.7), and in terms of a method for its identification.

In the present case, the claims so lack support, and the application so lacks disclosure, that a meaningful search over the whole of the claimed scope is impossible.

Independent of the above reasoning, the claims also lack clarity (Article 84 EPC). An attempt is made to define a compound by reference to a result to be achieved. Again, this lack of clarity in the present case is such as to render a meaningful search over the whole of the claimed scope impossible.

Consequently, the search has been carried out for those parts of the

European Patent Office

INCOMPLETE SEARCH
SHEET C

Application Number
EP 04 02 8251

claims which appear to be clear, supported and disclosed, namely those parts relating to compounds obtainable by means of antisense technology and antibodies.

## ANNEX TO THE EUROPEAN SEARCH REPORT ON EUROPEAN PATENT APPLICATION NO. EP 04 02 8251

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2005

| Patent document cited in search report | Publication date | Patent family member(s) | Publication date |
|---|---|---|---|
| WO 03083047 A | 09-10-2003 | AU 2003213606 A1 | 13-10-2003 |
| | | AU 2003216482 A1 | 16-09-2003 |
| | | AU 2003223207 A1 | 16-09-2003 |
| | | AU 2003224638 A1 | 16-09-2003 |
| | | CA 2448112 A1 | 12-12-2002 |
| | | CA 2449479 A1 | 12-12-2002 |
| | | CA 2460944 A1 | 01-05-2003 |
| | | EP 1402051 A2 | 31-03-2004 |
| | | EP 1402262 A2 | 31-03-2004 |
| | | EP 1438432 A2 | 21-07-2004 |
| | | JP 2004532638 T | 28-10-2004 |
| | | JP 2005514906 T | 26-05-2005 |
| | | JP 2005506844 T | 10-03-2005 |
| | | WO 02098889 A2 | 12-12-2002 |
| | | WO 02098468 A1 | 12-12-2002 |
| | | WO 02098891 A2 | 12-12-2002 |
| | | WO 02099068 A2 | 12-12-2002 |
| | | WO 03035831 A2 | 01-05-2003 |
| | | WO 03083047 A2 | 09-10-2003 |
| | | WO 03074662 A2 | 12-09-2003 |
| | | WO 03073823 A2 | 12-09-2003 |
| | | WO 03074674 A2 | 12-09-2003 |
| | | US 2003138431 A1 | 24-07-2003 |
| | | US 2003165966 A1 | 04-09-2003 |
| | | US 2003215849 A1 | 20-11-2003 |
| | | US 2003219795 A1 | 27-11-2003 |

EPO FORM P0459

For more details about this annex : see Official Journal of the European Patent Office, No. 12/82

SEQUENCE LISTING

<110>  Technische Universität München

<120>  Sly1 inhibitors in transplantation

<130>  K2605 EP S3

<160>  16

<170>  PatentIn version 3.1

<210>  1

<211>  1143

<212>  DNA

<213>  mus musculus

<400>  1

```
atgttgcgtc gcaaaccctc caacgccagt gacaaagagc ccactcagaa gaaaaagctc      60
tcacttcagc gctccagcag cttcaaggat tttgccaaat cgaagcccag ctcccccgtg     120
gtgagcgaga aagaatttaa cctggatgat aatattccag aagatgattc aggtgttctt     180
accccggagg attctgggaa gagtggcaaa aagttgggaa agaagtggag ggcagtcatt     240
tcccgaacca tgaacaggaa gatgggcaag atgatggtga aggccttgtc agaggagatg     300
ggagacacgc tggaagaggg ctcagcttct ccaacatctc cagactgcag cctggacagc     360
cccgggcctg agaagatggc cctggccttt actgagcagg aggagcgtga gccgccatct     420
ctcagccgcc agacatcaac aggcagtgaa ctctgtagcc ctggcccagg ctcgggaagc     480
ttcctagagg agtcaccggc tccccagtac acggggcctt tctgtgggcg tgcccgagtc     540
cacactgact tcactcccag cccttatgac cacgactcat tgaaactgca gaaaggagat     600
gtgatccaga tcgttgaaaa gccacctgtg ggcacatggc tgggcctgct caatggcaag     660
ctgggttctt tcaaattcat ctacgtggat gtgctacccg aggaggctgt gggacctgta     720
cgccccagtc gccgacagag caaaggcaag aggcccaagc ccaagactct gcatgaactg     780
```

ctggagcgca ttggcctaga ggaacacaca tccaccctac tgctcaatgg ctaccagacg 840

ctcgaagact tcaaagagct gcgggaaacg cacctcaatg agctgaacat catggaccca 900

caacaccggg ccaagctgct cacagctgca gagctgctac tggactatga cactggcagt 960

gaagaggctg aagagggtgc tgagagcagc caggaacccg tggcgcacac agtgtcagaa 1020

cccaaggtgg acatcccacg tgactccggc tgctttgagg gctcagagag tgggcgtgat 1080

gaggcagagc tggcaggcac agaagagcaa ctgcagggtc tctccctgtc tggggcacct 1140

tga 1143

<210> 2

<211> 380

<212> PRT

<213> mus musculus

<400> 2

Met Leu Arg Arg Lys Pro Ser Asn Ala Ser Asp Lys Glu Pro Thr Gln
1               5                   10                  15

Lys Lys Lys Leu Ser Leu Gln Arg Ser Ser Ser Phe Lys Asp Phe Ala
            20                  25                  30

Lys Ser Lys Pro Ser Ser Pro Val Val Ser Glu Lys Glu Phe Asn Leu
        35                  40                  45

Asp Asp Asn Ile Pro Glu Asp Asp Ser Gly Val Leu Thr Pro Glu Asp
    50                  55                  60

Ser Gly Lys Ser Gly Lys Lys Leu Gly Lys Lys Trp Arg Ala Val Ile
65                  70                  75                  80

Ser Arg Thr Met Asn Arg Lys Met Gly Lys Met Met Val Lys Ala Leu
                85                  90                  95

Ser Glu Glu Met Gly Asp Thr Leu Glu Glu Gly Ser Ala Ser Pro Thr
            100                 105                 110

Ser Pro Asp Cys Ser Leu Asp Ser Pro Gly Pro Glu Lys Met Ala Leu
        115                 120                 125

Ala Phe Thr Glu Gln Glu Glu Arg Glu Pro Pro Ser Leu Ser Arg Gln
130 135 140

Thr Ser Thr Gly Ser Glu Leu Cys Ser Pro Gly Pro Gly Ser Gly Ser
145 150 155 160

Phe Leu Glu Glu Ser Pro Ala Pro Gln Tyr Thr Gly Pro Phe Cys Gly
165 170 175

Arg Ala Arg Val His Thr Asp Phe Thr Pro Ser Pro Tyr Asp His Asp
180 185 190

Ser Leu Lys Leu Gln Lys Gly Asp Val Ile Gln Ile Val Glu Lys Pro
195 200 . 205

Pro Val Gly Thr Trp Leu Gly Leu Leu Asn Gly Lys Leu Gly Ser Phe
210 215 220

Lys Phe Ile Tyr Val Asp Val Leu Pro Glu Glu Ala Val Gly Pro Val
225 230 235 240

Arg Pro Ser Arg Arg Gln Ser Lys Gly Lys Arg Pro Lys Pro Lys Thr
245 250 255

Leu His Glu Leu Leu Glu Arg Ile Gly Leu Glu Glu His Thr Ser Thr
260 265 270

Leu Leu Leu Asn Gly Tyr Gln Thr Leu Glu Asp Phe Lys Glu Leu Arg
275 280 285

Glu Thr His Leu Asn Glu Leu Asn Ile Met Asp Pro Gln His Arg Ala
290 295 300

Lys Leu Leu Thr Ala Ala Glu Leu Leu Leu Asp Tyr Asp Thr Gly Ser
305 310 315 320

Glu Glu Ala Glu Glu Gly Ala Glu Ser Ser Gln Glu Pro Val Ala His
325 330 335

Thr Val Ser Glu Pro Lys Val Asp Ile Pro Arg Asp Ser Gly Cys Phe
340 345 350

Glu Gly Ser Glu Ser Gly Arg Asp Glu Ala Glu Leu Ala Gly Thr Glu
355 360 365

Glu Gln Leu Gln Gly Leu Ser Leu Ser Gly Ala Pro
370 375 380

<210> 3

<211> 1143

<212> DNA

<213> homo sapiens

<400> 3
atgctgcgcc gcaagccctc caatgccagt gagaaggagc ccactcagaa gaaaaagctc 60

tcccttcagc gctccagcag cttcaaggat tttgccaaat ccaaacccag ctcccccgtg 120

gtgagcgaga aggagtttaa tctggatgat aacattccag aagatgactc aggtgtcccc 180

accccagaag atgctgggaa gagtggcaaa aagctgggga agaagtggag ggcagtgatt 240

tcccgaacca tgaacaggaa gatgggcaag atgatggtga aggccctgtc agaagagatg 300

gcagacactc tggaggaggg ctctgcctcc ccgacatctc cagactacag cctggacagc 360

cctggccctg agaagatggc gctggccttt tctgagcaag aggagcatga acttccggtg 420

ctcagccgcc aggcatcaac aggcagtgag ctctgcagcc ccagcccagg ttctggcagc 480

ttcggggagg aaccacctgc cccccagtac acagggcctt tctgtggccg ggcacgagtc 540

cacaccgact tcactcccag cccctatgac cacgactcgc tgaaactgca gaaaggagat 600

gtgatccaga tcattgaaaa gccacctgtg ggcacgtggc tgggcctact caatggcaag 660

gtgggctctt tcaaattcat ctatgtggat gtgctgcccg aggaggccgt ggggcatgcc 720

cgccccagcc gccgacagag caagggcaag aggcccaagc ctaagaccct gcatgagctg 780

ctggagcgca tcggcctgga ggagcacaca tccaccctcc tgctcaatgg ctaccagaca 840

ctggaagact tcaaagagct gcgagaaaca cacctcaatg agctgaacat catggatcca 900

cagcaccggg ccaagctgct cacggccgcc gagctgctgc tggactatga cactggcagt 960

gaggaggctg aagagggcgc cgagagcagc caggagccag tggcacacac agtgtcggaa 1020

cccaaggtgg acatcccgcg cgactcaggc tgctttgagg gctcggagag cgggcgcgat 1080

gacgcagagc tggcaggcac tgaggagcag ctgcaaggcc tctccctggc cggggcacct 1140

tga 1143

<210> 4

<211> 380

<212> PRT

<213> homo sapiens

<400> 4

Met Leu Arg Arg Lys Pro Ser Asn Ala Ser Glu Lys Glu Pro Thr Gln
1 5 10 15

Lys Lys Lys Leu Ser Leu Gln Arg Ser Ser Ser Phe Lys Asp Phe Ala
20 25 30

Lys Ser Lys Pro Ser Ser Pro Val Val Ser Glu Lys Glu Phe Asn Leu
35 40 45

Asp Asp Asn Ile Pro Glu Asp Asp Ser Gly Val Pro Thr Pro Glu Asp
50 55 60

Ala Gly Lys Ser Gly Lys Lys Leu Gly Lys Lys Trp Arg Ala Val Ile
65 70 75 80

Ser Arg Thr Met Asn Arg Lys Met Gly Lys Met Met Val Lys Ala Leu
85 90 95

Ser Glu Glu Met Ala Asp Thr Leu Glu Glu Gly Ser Ala Ser Pro Thr
100 105 110

Ser Pro Asp Tyr Ser Leu Asp Ser Pro Gly Pro Glu Lys Met Ala Leu
115 120 125

Ala Phe Ser Glu Gln Glu Glu His Glu Leu Pro Val Leu Ser Arg Gln

130 135 140

Ala Ser Thr Gly Ser Glu Leu Cys Ser Pro Ser Pro Gly Ser Gly Ser
145 150 155 160

Phe Gly Glu Glu Pro Pro Ala Pro Gln Tyr Thr Gly Pro Phe Cys Gly
165 170 175

Arg Ala Arg Val His Thr Asp Phe Thr Pro Ser Pro Tyr Asp His Asp
180 185 190

Ser Leu Lys Leu Gln Lys Gly Asp Val Ile Gln Ile Ile Glu Lys Pro

```
        195                 200                 205

Pro Val Gly Thr Trp Leu Gly Leu Leu Asn Gly Lys Val Gly Ser Phe
    210                 215                 220

Lys Phe Ile Tyr Val Asp Val Leu Pro Glu Glu Ala Val Gly His Ala
225                 230                 235                 240

Arg Pro Ser Arg Arg Gln Ser Lys Gly Lys Arg Pro Lys Pro Lys Thr
                245                 250                 255

Leu His Glu Leu Leu Glu Arg Ile Gly Leu Glu Glu His Thr Ser Thr
            260                 265                 270

Leu Leu Leu Asn Gly Tyr Gln Thr Leu Glu Asp Phe Lys Glu Leu Arg
        275                 280                 285

Glu Thr His Leu Asn Glu Leu Asn Ile Met Asp Pro Gln His Arg Ala
    290                 295                 300

Lys Leu Leu Thr Ala Ala Glu Leu Leu Leu Asp Tyr Asp Thr Gly Ser
305                 310                 315                 320

Glu Glu Ala Glu Glu Gly Ala Glu Ser Ser Gln Glu Pro Val Ala His
                325                 330                 335

Thr Val Ser Glu Pro Lys Val Asp Ile Pro Arg Asp Ser Gly Cys Phe
            340                 345                 350

Glu Gly Ser Glu Ser Gly Arg Asp Asp Ala Glu Leu Ala Gly Thr Glu
        355                 360                 365

Glu Gln Leu Gln Gly Leu Ser Leu Ala Gly Ala Pro
    370                 375                 380

<210>  5

<211>  1146

<212>  DNA

<213>  rattus norvegicus


<400>  5
atgttgcgtc gcaaaccctc caacgctagt gacaaggagc ctactcagaa aaaaaagctc      60
```

```
tcacttcagc gctccagcag cttcaaggac tttgccaaat cgaagcccag ctcccctgtg     120
gtgagcgaga aagaatttaa cctggatgat aatattccag aagatgactc aggtgttctt     180
accccagagg attctgggaa gagtggcaaa aagttgggaa agaagtggag ggcagtcatt     240
tccagaacca tgaacaggaa gatgggcaag atgatggtga aggccctgtc agaggagatg     300
ggagacacgc tggaggaggg ttcagcttcc ccaacatctc cagactgcag cctggacagc     360
cctggtcctg agaaaatggc cctggccttt tctgagccag aggagcgtga gctgctagct     420
ctcagccgcc agacatcaac aggcagtgaa ctctgtagcc ctggcccagg ctctggaagc     480
ttcatagagg agtcacctgc tccccagtac acggggcctt tctgtgggcg tgcccgagtc     540
cacacagact tcactcccag cccttatgac cacgattcat tgaaactgca gaaaggagat     600
gtgatccaga tcattgaaaa gccacccgtg ggcacatggt tgggcctgct gaacggcaag     660
ctaggctctt tcaaattcat ctacgtggat gtgctacctg aggaggccgt ggggcctgta     720
cgccccagtc gccgacagag caaaagcaag agacccaagc ccaagactct gcatgaactg     780
ctggagcgca ttggcctgga ggagcacaca tccaccctgc tgctcaatgg ctaccagaca     840
ctggaagact tcaaagagct gcgggaaaca cacctcaatg agctcaacat catggaccct     900
cagcaccggg ccaagctgct cacagctgca gagctgctgc tggactatga cacagctggc     960
agtgaagagg ccgaagaggg cgctgagagc agccaggagc ccgcgctgca cacagtgtca    1020
gaacccaagg tggacatccc acgtgactct ggctgctttg agggctcaga gagtggccgt    1080
gatgaggcgg agctgacagg cacagaggag cagctacagg gtctctccct ggctggggca    1140
ccatga                                                               1146
```

<210> 6

<211> 381

<212> PRT

<213> rattus norvegicus

<400> 6

```
Met Leu Arg Arg Lys Pro Ser Asn Ala Ser Asp Lys Glu Pro Thr Gln
1               5                   10                  15

Lys Lys Lys Leu Ser Leu Gln Arg Ser Ser Ser Phe Lys Asp Phe Ala
            20                  25                  30

Lys Ser Lys Pro Ser Ser Pro Val Val Ser Glu Lys Glu Phe Asn Leu
```

35 40 45

Asp Asp Asn Ile Pro Glu Asp Asp Ser Gly Val Leu Thr Pro Glu Asp
50 55 60

Ser Gly Lys Ser Gly Lys Lys Leu Gly Lys Lys Trp Arg Ala Val Ile
65 70 75 80

Ser Arg Thr Met Asn Arg Lys Met Gly Lys Met Met Val Lys Ala Leu
85 90 95

Ser Glu Glu Met Gly Asp Thr Leu Glu Glu Gly Ser Ala Ser Pro Thr
100 105 110

Ser Pro Asp Cys Ser Leu Asp Ser Pro Gly Pro Glu Lys Met Ala Leu
115 120 125

Ala Phe Ser Glu Pro Glu Glu Arg Glu Leu Leu Ala Leu Ser Arg Gln
130 135 140

Thr Ser Thr Gly Ser Glu Leu Cys Ser Pro Gly Pro Gly Ser Gly Ser
145 150 155 160

Phe Ile Glu Glu Ser Pro Ala Pro Gln Tyr Thr Gly Pro Phe Cys Gly
165 170 175

Arg Ala Arg Val His Thr Asp Phe Thr Pro Ser Pro Tyr Asp His Asp
180 185 190

Ser Leu Lys Leu Gln Lys Gly Asp Val Ile Gln Ile Ile Glu Lys Pro
195 200 205

Pro Val Gly Thr Trp Leu Gly Leu Leu Asn Gly Lys Leu Gly Ser Phe
210 215 220

Lys Phe Ile Tyr Val Asp Val Leu Pro Glu Glu Ala Val Gly Pro Val
225 230 235 240

Arg Pro Ser Arg Arg Gln Ser Lys Ser Lys Arg Pro Lys Pro Lys Thr
245 250 255

Leu His Glu Leu Leu Glu Arg Ile Gly Leu Glu Glu His Thr Ser Thr
260 265 270

Leu Leu Leu Asn Gly Tyr Gln Thr Leu Glu Asp Phe Lys Glu Leu Arg

275 280 285

Glu Thr His Leu Asn Glu Leu Asn Ile Met Asp Pro Gln His Arg Ala
290 295 300

Lys Leu Leu Thr Ala Ala Glu Leu Leu Leu Asp Tyr Asp Thr Ala Gly
305 310 315 320

Ser Glu Glu Ala Glu Glu Gly Ala Glu Ser Ser Gln Glu Pro Ala Leu
325 330 335

His Thr Val Ser Glu Pro Lys Val Asp Ile Pro Arg Asp Ser Gly Cys
340 345 350

Phe Glu Gly Ser Glu Ser Gly Arg Asp Glu Ala Glu Leu Thr Gly Thr
355 360 365

Glu Glu Gln Leu Gln Gly Leu Ser Leu Ala Gly Ala Pro
370 375 380

<210> 7

<211> 19

<212> DNA

<213> artificial sequence

<220>

<223> antisense molecule

<400> 7
gcaagaggcc tctgcatct 19

<210> 8

<211> 19

<212> DNA

<213> artificial sequence

<220>

<223> antisense molecule

<400> 8

gccctccaat gccagtgag 19

<210> 9

<211> 19

<212> DNA

<213> artificial sequence

<220>

<223> antisense molecule

<400> 9
ggagcccact cagaagaaa 19

<210> 10

<211> 19

<212> DNA

<213> artificial sequence

<220>

<223> antisense molecule

<400> 10
agctctccct tcagcgctc 19

<210> 11

<211> 19

<212> DNA

<213> artificial sequence

<220>

<223> antisense molecule

<400> 11
gatgctggga agagtggca 19

<210> 12

<211> 19

<212> DNA

<213> artificial sequence

<220>

<223> antisense molecule

<400> 12
ggcctccaaa tcctcctca 19

<210> 13

<211> 19

<212> DNA

<213> artificial sequence

<220>

<223> antisense molecule

<400> 13
gaacccaaac tgaccatgg 19

<210> 14

<211> 19

<212> DNA

<213> artificial sequence

<220>

<223> antisense molecule

<400> 14
atccacaggt accattccc 19

<210> 15

<211> 19

<212> DNA

<213> artificial sequence

<220>

<223> antisense molecule

<400> 15
atggctcctc ctactcacc 19

<210> 16

<211> 19

<212> DNA

<213> artificial sequence

<220>

<223> antisense molecule

<400> 16
gccagctgtg gatctgagt 19